# EUROPEAN PATENT SPECIFICATION

(11) **EP 0 949 930 B1**
(45) Date of publication and mention of the grant of the patent: **06.10.2004**
(21) Application number: 97933154.3
(22) Date of filing: 19.06.1997
(51) Int. Cl.: A61K 38/46, C07H 19/00, C07H 21/02, C07H 21/04, C12N 9/14, C12N 1/20, C12N 15/00

(54) **THERMOSTABLE PHOSPHATASES**
THERMOSTABILEN PHOSPHATASEN
PHOSPHATASES THERMOSTABLES

(30) Priority: 19.06.1996 US 33752 P
(43) Date of publication of application: 20.10.1999
(73) Proprietor: Diversa Corporation, San Diego, CA 92121 (US)
(72) Inventor: MATHUR, Eric, J., Carlsbad, CA 92009 (US); LEE, Edd, La Jolla, CA 92037 (US); BYLINA, Edward, Andalusia, PA 19020 (US)
(74) Representative: VOSSIUS & PARTNER
(86) International application number: PCT/US1997/010784
(87) International publication number: WO 1997/048416

(56) References cited:
- EP-A- 0 866 129
- WO-A-95/30756
- WO-A-96/37612
- DATABASE WPI Section Ch, Week 199342 Derwent Publications Ltd., London, GB; Class B04, AN 1993-330578 XP002144498 & JP 05 236955 A (UNITIKA LTD), 17 September 1993 (1993-09-17)
- EMBL DATABASE Accession no D83525 Sequence identity PSCS 29 February 1996 RAHAMAN N ET AL: "Gene cloning and sequence ananlysis of cobyric acid synthase and cobalamin (5'-phosphate) synthase from hyperthermophilic archaeon Pyrococcus spec." XP002151693
- BULT CAROL J ET AL: "Complete genome sequence of the methanogenic archaeon, Methanococcus jannaschii." SCIENCE (WASHINGTON D C), vol. 273, no. 5278, 23 August 1996 (1996-08-23), pages 1058-1073, XP002151692 ISSN: 0036-8075 -& EMBL DATABASE Accession no U67505 Sequence identitity MJU67505 26 August 1996 XP002151694
- EMBL DATABASE Accession no AA080579, Sequence identity SSAA80579 16 October 1996 CARSON D ET AL: "Sugercane cDNA from leaf roll tissue" XP002151695
- CLINICAL CHEMISTRY, December 1992, Vol. 38, No. 12, MOSS, "Perspectives in Alkaline Phosphatase Research", pages 2486-2492.
- CRITICAL REVIEWS IN CLINICAL LABORATORY SCIENCES, July 1995, Vol. 32, No. 4, MOSS et al., "Clinical and Biological Aspects of Acid Phosphatase", pages 431-467.

## Description

This invention relates to newly identified polynucleotides, polypeptides encoded by such polynucleotides, the use of such polynucleotides and polypeptides, as well as the production and isolation of such polynucleotides and polypeptides. More particularly, the polynucleotides and polypeptides of the present invention have been identified as thermostable alkaline phosphatases from Thermococcus.

### BACKGROUND OF THE INVENTION

Phosphatases are a group of enzymes that remove phosphate groups from organophosphate ester compounds. There are numerous phosphatases, including alkaline phosphatases, phosphodiesterases and phytases.

Alkaline phosphatases are widely distributed enzymes and are composed of a group of enzymes which hydrolyze organic phosphate ester bonds at alkaline pH.

Phosphodiesterases are capable of hydrolyzing nucleic acids by hydrolyzing the phosphodiester bridges of DNA and RNA. The classification of phosphodiesterases depends upon which side of the phosphodiester bridge is attacked. The 3' enzymes specifically hydrolyze the ester linkage between the 3' carbon and the phosphoric group whereas the 5' enzymes hydrolyze the ester linkage between the phosphoric group and the 5' carbon of the phosphodiester bridge. The best known of the class 3' enzymes is a phosphodiesterase from the venom of the rattlesnake or from a rustle's viper, which hydrolyses all the 3' bonds in either RNA or DNA liberating nearly all the nucleotide units as nucleotide 5' phosphates. This enzyme requires a free 3' hydroxyl group on the terminal nucleotide residue and proceeds stepwise from that end of the polynucleotide chain. This enzyme and all other nucleases which attack only at the ends of the polynucleotide chains are called exonucleases. The 5' enzymes are represented by a phosphodiesterase from bovine spleen, also an exonuclease, which hydrolyses all the 5' linkages of both DNA and RNA and thus liberates only nucleoside 3' phosphates. It begins its attack at the end of the chain having a free 3' hydroxyl group.

Phytases are enzymes which recently have been introduced to commerce. The phytase enzyme removes phosphate from phytic acid (inositol hexaphosphoric acid), a compound found in plants such as corn, wheat and rice. The enzyme has commercial use for the treatment of animal feed, making the inositol of the phytic acid available for animal nutrition. *Aspergillus ficuum* and wheat are sources of phytase. (Business Communications Co., Inc., 25 Van Zant Street, Norwalk, CT 06855).

Phytase is used to improve the utilization of natural phosphorus in animal feed. Use of phytase as a feed additive enables the animal to metabolize a larger degree of its cereal feed's natural mineral content thereby reducing or altogether eliminating the need for synthetic phosphorus additives. More important than the reduced need for phosphorus additives is the corresponding reduction of phosphorus in pig and chicken waste. Many European countries severely limit the amount of manure that can be spread per acre due to concerns regarding phosphorus contamination of ground water. This is highly important in northern Europe, and will eventually be regulated throughout the remainder of the European Continent and the United States as well. (Excerpts from Business Trend Analysts, Inc., January 1994, Frost and Sullivan Report 1995 and USDA on-line information.)

Alkaline phosphatase hydrolyzes monophosphate esters, releasing an organic phosphate and the cognate alcohol compound. It is non-specific with respect to the alcohol moiety and it is this feature which accounts for the many uses of this enzyme. The enzyme has a pH optimum between 9 and 10, however, it can also function at neutral pH, (study of the enzyme industry conducted by Business Communications Company, Inc., 25 Van Zant Street, Norwalk, Connecticut 06855, 1995.).

Thermostable alkaline phosphatases are not irreversibly inactivated even when heated to 60°C or more for brief periods of time, as, for example, in the practice of hydrolyzing monophosphate esters.

Alkaline phosphatases may be obtained from numerous thermophilic organisms, such as *Ammonifex degensii, Aquifex pyrophilus, Archaeoglobus lithotrophicus, Methanococcus igneus, Pyrolobus*(a *Crenarchaeota*), *Pyrococcus* and *Thermococcus*, which are mostly Eubacteria and Euryarchaeota. Many of these organisms grow at temperatures up to about 103°C and are unable to grow below 70°C. These anaerobes are isolated from extreme environments. For example, *Thermococcus* CL-2 was isolated from a worm residing on a "black smoker" sulfite structure.

Interest in alkaline phosphatases from thermophilic microbes has increased recently due to their value for commercial applications. Two sources of alkaline phosphatases dominate and compete commercially: (i) animal, from bovine and calf intestinal mucosa, and (ii) bacterial, from E. coli. Due to the high turnover number of calf intestinal phosphatase, it is often selected as the label in many enzyme immunoassays. The usefulness of calf alkaline phosphatase, however, is limited by its inherently low thermostability, which is even further compromised during the chemical preparation of the enzyme: antibody conjugates. Bacterial alkaline phosphatase is an alternative to calf alkaline phosphatase due to bacterial alkaline phosphatase's extreme thermotolerance at temperatures as high as 95°C (Tomazic-Allen, S.J., Recombinant Bacterial Phosphatase as an Immunodiagnostic Enzyme, Annals D Biology Clinique, 49(5):287-90 (1991), however, the enzyme has a very low turnover number.

There is a need for novel phosphatase enzymes having enhanced thermostability. This includes a need for thermostable alkaline phosphatases whose enhanced thermostability is beneficial in enzyme labeling processes and certain recombinant DNA techniques, such as in the dephosphorylation of vector DNA prior to insert DNA ligation. Recombinant phosphatase enzymes provide the proteins in a format amenable to efficient production of pure enzyme, which can be utilized in a variety of applications as described herein. Accordingly, there is a need for the characterization, amino acid sequencing, DNA sequencing, and heterologous expression of thermostable phosphatase enzymes. The present invention meets these need by providing DNA and amino acid sequence information and exprssion and purification protocol for thermostable phosphatase derived from several organisms.

### SUMMARY OF THE INVENTION

The present invention provides thermostable phosphatases from Thermococcus. In accordance with one aspect of the present invention, there are provided novel enzymes, as well as active fragments, analogs and derivatives thereof.

In accordance with another aspect of the present invention, there are provided isolated nucleic acid molecules encoding the enzymes of the present invention, including mRNAs, cDNAs, genomic DNAs, as well as active analogs and fragments of such nucleic acids.

In accordance with another aspect of the present invention, there are provided isolated nucleic acid molecules encoding mature enzymes expressed by the DNA contained in the plasmid DNA vector deposited with the ATCC as Deposit No. 97536 on May 10, 1996.

In accordance with a further aspect of the present invention, there is provided a process for producing such polypeptides by recombinant techniques comprising culturing recombinant prokaryotic and/or eukaryotic host cells, containing a nucleic acid sequence of the present invention, under conditions promoting expression of said enzymes and subsequent recovery of said enzymes.

In accordance with yet a further aspect of the present invention, there is provided a process for utilizing such enzymes for hydrolyzing monophosphate ester bonds, as an enzyme label in immunoassays, for removing 5' phosphate prior to end-labeling, and for dephosphorylating vectors prior to insert ligation.

In accordance with yet a further aspect of the present invention, there are also provided nucleic acid probes comprising nucleic acid molecules of sufficient length to specifically hybridize to a nucleic acid sequence of the present invention.

In accordance with yet a further aspect of the present invention, there is provided a process for utilizing such enzymes, or polynucleotides encoding such enzymes, for in vitro purposes related to scientific research, for example, to generate probes for identifying similar sequences which might encode similar enzymes from other organisms by using certain regions, *i.e*., conserved sequence regions of the nucleotide sequence.

These and other aspects of the present invention will be apparent to those of skill in the art from the teachings herein.

### BRIEF DESCRIPTION OF THE DRAWINGS

The following drawings show DNA sequences described in the specification herein below.
Figure 1 is an illustration of the full-length-DNA and corresponding deduced amino acid sequence of *Ammonifex degensii* KC4 of the present invention. Sequencing was performed using a 378 automated DNA sequence for all sequences of the present. invention (Applied Biosystems, Inc., Foster City, California).
Figure 2 is an illustration of the full-length DNA and corresponding deduced amino acid sequence of *Methanococcus igneus* Ko15.
Figure 3 is an illustration of the full-length DNA and corresponding deduced amino acid sequence of *Thermococcus alcaliphilus* AEDII12RA.
Figure 4 is an illustration of the full-length DNA and corresponding deduced amino acid sequence of *Thermococcus celer*.
Figure 5 is an illustration of the full-length DNA and corresponding deduced amino acid sequence of *Thermococcus* GU5L5.
Figure 6 is an illustration of the full-length DNA and corresponding deduced amino acid sequence of OC9a.
Figure 7 is an illustration of the full-length DNA and corresponding deduced amino acid sequence of M11TL.
Figure 8 is an illustration of the full-length DNA and corresponding deduced amino acid sequence of *Thermococcus* CL-2.
Figure 9 is an illustration of the full-length DNA and corresponding deduced amino acid sequence of *Aquifex* VF-5.

### DETAILED DESCRIPTION OF THE INVENTION

To facilitate understanding of the invention, a number of terms are defined below.

The term "isolated" means altered "by the hand of man" from its natural state; i.e., if it occurs in nature, it has been changed or removed from its original environment, or both. For example, a naturally occurring polynucleotide or a polypeptide naturally present in a living animal in its natural state is not "isolated", but the same polynucleotide or polypeptide separated from the coexisting materials of its natural state is "isolated", as the term is employed herein. For example, with respect to polynucleotides, the term isolated means that it is separated from the nucleic acid and cell in which it naturally occurs.

As part of or following isolation, such polynucleotides can be joined to other polynucleotides, such as DNAs, for mutagenesis, to form fusion proteins, and for propagation or expression in a host, for instance. The isolated polynucleotides, alone or joined to other polynucleotides such as vectors, can be introduced into host cells, in culture or in whole organisms. Introduced into host cells in culture or in whole organisms, such polynucleotides still would be isolated, as the term is used herein, because they would not be in their naturally occurring form or environment. Similarly, the polynucleotides and polypeptides may occur in a composition, such as a media formulation (solutions for introduction of polynucleotides or polypeptides, for example, into cells or compositions or solutions for chemical or enzymatic reactions which are not naturally occurring compositions) and, therein remain isolated polynucleotides or polypeptides within the meaning of that term as it is employed herein.

The term "ligation" refers to the process of forming phosphodiester bonds between two or more polynucleotides, which most often are double stranded DNAs. Techniques for ligation are well known to the art and protocols for ligation are described in standard laboratory manuals and references, such as, for instance, Sambrook et al., MOLECULAR CLONING, A LABORATORY MANUAL, 2nd Ed.; Cold Spring Harbor Laboratory Press, Cold Spring Harbor, New York (1989).

The term "oligonucleotide" as used herein is defined as a molecule comprised of two or more deoxyribonucleotides or ribonucleotides, preferably more than three, and usually more than ten. The exact size of an oligonucleotide will depend on many factors, including the ultimate function or use of the oligonucleotide. Oligonucleotides can be prepared by any suitable method, including, for example, cloning and restriction of appropriate sequences and direct chemical synthesis by a method such as the phosphotriester method of Narang *et al*., 1979, Meth. Enzymol., 68:90-99; the phosphodiester method of Brown et al., 1979, Method Enzymol., 68:109-151, the diethylphosphoramidite method of Beaucage *et al*., 1981, Tetrahedron Lett., 22:1859-1862; the triester method of Matteucci *et al*., 1981, J. Am. Chem. Soc., 103:3185-3191, or automated synthesis methods; and the solid support method of U.S. Patent No. 4,458,066.

The term "plasmids" generally is designated herein by a lower case p preceded and/or followed by capital letters and/or numbers, in accordance with standard naming conventions that are familiar to those of skill in the art.

Plasmids disclosed herein are either commercially available, publicly available on an unrestricted basis, or can be constructed from available plasmids by routine application of well known, published procedures. Many plasmids and other cloning and expression vectors that can be used in accordance with the present invention are well known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

The term "polynucleotide(s)" generally refers to any polyribonucleotide or polydeoxyribonucleotide, which may be unmodified RNA or DNA or modified RNA or DNA. Thus, for instance, polynucleotides as used herein refers to, among others, single-and double-stranded DNA, DNA that is a mixture of single-and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions.

In addition, polynucleotide as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide.

As used herein, the term polynucleotide includes DNAs or RNAs as described above that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritylated bases, to name just two examples, are polynucleotides as the term is used herein.

It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including simple and complex cells, *inter alia*.

The term "primer" as used herein refers to an oligonucleotide, whether natural or synthetic, which is capable of acting as a point of initiation of synthesis when placed under conditions in which primer extension is initiated or possible. Synthesis of a primer extension product which is complementary to a nucleic acid strand is initiated in the presence of nucleoside triphosphates and a polymerase in an appropriate buffer at a suitable temperature.

The term "primer" may refer to more than one primer, particularly in the case where there is some ambiguity in the information regarding one or both ends of the target region to be synthesized. For instance, if a nucleic acid sequence is inferred from a protein sequence, a "primer" generated to synthesize nucleic acid encoding said protein sequence is actually a collection of primer oligonucleotides containing sequences representing all possible codon variations based on the degeneracy of the genetic code. One or more of the primers in this collection will be homologous with the end of the target sequence. Likewise, if a "conserved" region shows significant levels of polymorphism in a population, mixtures of primers can be prepared that will amplify adjacent sequences.

The term "restriction endonucleases" and "restriction enzymes" refers to bacterial enzymes which cut double-stranded DNA at or near a specific nucleotide sequence.

The term "gene" means the segment of DNA involved in producing a polypeptide chain;
it includes regions preceding and following the coding region (leader and trailer) as well as intervening sequences (introns) between individual coding segments (exons).

A coding sequence is "operably linked" to another coding sequence when RNA polymerase will transcribe the two coding sequences into a single mRNA, which is then translated into a single polypeptide having amino acids derived from both coding sequences. The coding sequences need not be contiguous to one another so long as the expressed sequences ultimately process to produce the desired protein.

"Recombinant" enzymes refer to enzymes produced by recombinant DNA techniques; *i.e*., produced from cells transformed by an exogenous DNA construct encoding the desired enzyme. "Synthetic" enzymes are those prepared by chemical synthesis.

A DNA "coding sequence of" or a "nucleotide sequence encoding" a particular enzyme, is a DNA sequence which is transcribed and translated into an enzyme when placed under the control of appropriate regulatory sequences.

The term "thermostable phosphatase" refers to an enzyme which is stable to heat and heat-resistant and catalyzes the removal of phosphate groups from organophosphate ester compounds. Reference to "thermostable phosphatases" includes alkaline phosphatases, phosphodiesterases and phytases.

The phosphatase enzymes of the present invention cannot become irreversibly denatured (inactivated) when subjected to the elevated temperatures for the time necessary to effect the hydrolysis of a phosphate group from an organophosphate ester compound. Irreversible denaturation for purposes herein refers to permanent and complete loss of enzymatic activity. The phosphatase enzymes do not become irreversibly denatured from exposure to temperatures of a range from about 60°C to about 113°C or more. The extreme thermostability of the phosphatase enzymes provides additional advantages over previously characterized thermostable enzymes. Prior to the present invention, efficient hydrolysis of phosphate groups at temperatures as high as 100°C has not been demonstrated. No thermostable phosphatase has been described for this purpose.

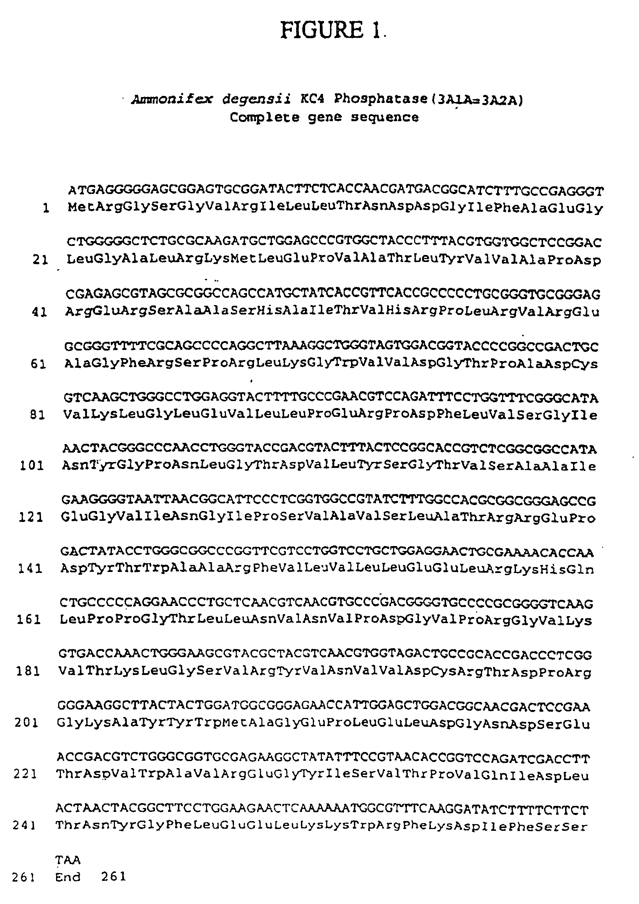

The specification also describes isolated nucleic acids (polynucleotides) which encode for the mature enzymes having the deduced amino acid sequences of Figures 1-9 (SEQ ID NOS: 28-36).

In accordance with another aspect of the present invention, there are provided isolated polynucleotides encoding the enzymes of the present invention. The deposited material is a mixture of genomic clones comprising DNA encoding an enzyme of the present invention. Each genomic clone comprising the respective DNA has been inserted into a pBluescript vector (Stratagene, La Jolla, CA). The deposit has been deposited with the American Type Culture Collection, 12301 Parklawn Drive, Rockville, Maryland 20852, USA, on May 10, 1996 and assigned ATCC Deposit No. 97536.

The deposit(s) have been made under the terms of the Budapest Treaty on the International Recognition of the deposit of micro-organisms for purposes of patent procedure. The strains will be irrevocably and without restriction or condition released to the public upon the issuance of a patent. These deposits are provided merely as convenience to those of skill in the art and are not an admission that a deposit be required under 35 U.S.C. §112. The sequences of the polynucleotides contained in the deposited materials, as well as the amino acid sequences of the polypeptides encoded thereby, are controlling in the event of any conflict with any description of sequences herein. A license may be required to make, use or sell the deposited materials, and no such license is hereby granted.

The polynucleotides of this invention were originally recovered from genomic gene libraries derived from the following organisms:

*Thermococcus alcaliphilus* AEDII12RA is from the genus *Thermococcus*. AEDII12RA grows optimally at 85°C, pH 9.5 in a high salt medium (marine) containing polysulfides and yeast extract as substrates and N₂ in gas phase.

*Thermococcus celer* is an *Euryarchaeota.* It grows optimally at 85°C and pH 6.0 in a high-salt marine medium containing elemental sulfur, yeast extract, and peptone as substrates and N₂ in gas phase.

*Thermococcus* GU5L5 is an *Euryarchaeota* isolated from the Guaymas Basin in Mexico. It grows optimally at 85°C and pH 6.0 in a high-salt marine medium containing 1% elemental sulfur, 0.4% yeast extract, and 0.5% peptone as substrates with N₂ in gas phase.

*Thermococcus* CL-2 is an *Euryarchaeota* isolated from the North Cleft Segment in the Juan de Fuca Ridge. It grows optimally at 88°C in a salt medium with an argon atmosphere.

Other polynucleotides, not part of this invention were originally recovered from genomic gene libraries derived from the following organisms:

*Ammonifex degensii* KC4 is a eubacteria from the genus *Ammonifex.* It was isolated in Java, Indonesia. It is a gram-negative, chemolithoautotroph. It grows optimally at 70°C in a low-salt culture medium at pH 7 with 0.2% nitrate as a substrate and H₂/CO₂ in gas phase.

*Methanococcus igneus* KOL5 is a *Euryarchaeota* isolated from Kolbeinsey Ridge in the north of Iceland. It grows optimally at 85°C and pH 7.0 in a high-salt marine medium with H₂/CO₂ in a gas phase. *Aquifex pyrophilus* KOL 5A is a marine bacteria isolated from th Kolbeinsey Ridge in the north of Iceland. It is a gram-negative, rod-shaped, strictly chemolithoautotrophic, knall gas bacterium, and a denitrifier. It grows optimally at 85°C in high-salt marine medium at pH 6.8 with O₂ as a substrate and H₂/CO₂ + 0.5% O₂ in gas phase.

OC9a-27A3A is a bacteria of unknown etilogy obtained from Yellowstone National Park and maintained as a pure culture. It grows well on a TK6 medium and has cellulose degrader activity. Further, it codes for an alkaline phosphatase having greater than 50% polypeptide identity and greater than 32% polynucleotide identity to each of *Bombyx* mori and *Escherichia coli C* alkaline phosphatase precursors, which is significant homoloygy. Thus, it is expectged that *OC9a*-27A3A can be cloned and expressed readily in *Escherichi Coli C* in place of its native alkaline phosphatase precursor.

M11 TL is a new species of *Desulfurococcus* isolated from Diamond Pool in Yellowstone National Park. M11TL grows heterotrophically by fermentation of different organic materials (sulfur is not necessary) and forms grape-like aggregates. The organism grows optimally at 85°C to 88°C and pH 7.0 in a low salt medium containing yeast extract, peptone, and gelatin as substrates with an N₂/CO₂ gas phase.

*Aquifex* VF-5 is a marine bacteria isolated from a beach in Vulcano, Italy. It is a gram-negative, rod-shaped, strictly chemolithoautotrophic, knall gas bacterium. It grows optimally from 85-90°C in high-salt marine medium at pH 6.8, with O₂ as a substrate and H₂/CO₂ + 0.5% O₂ in gas phase.

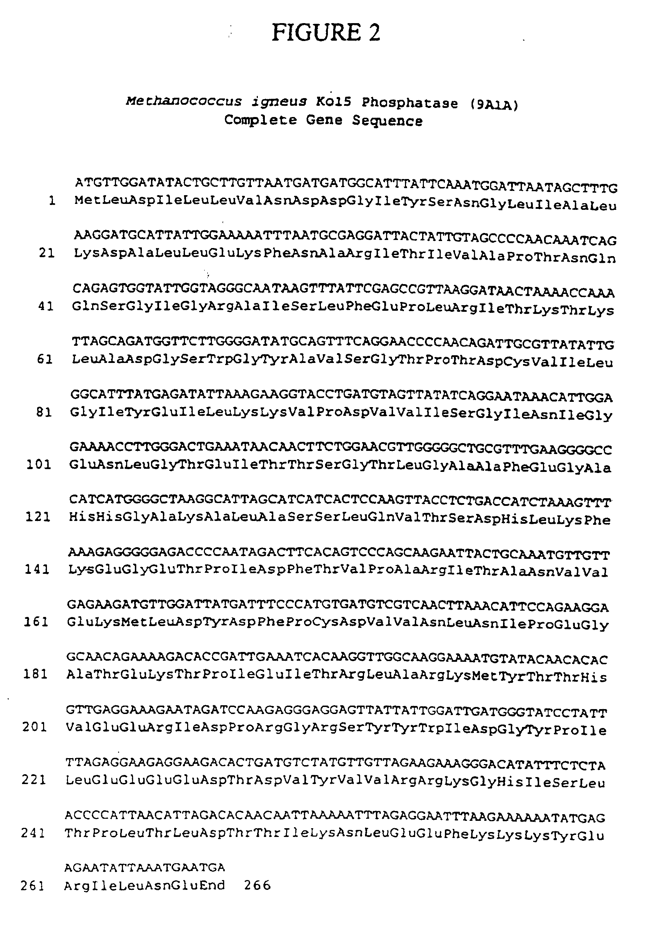

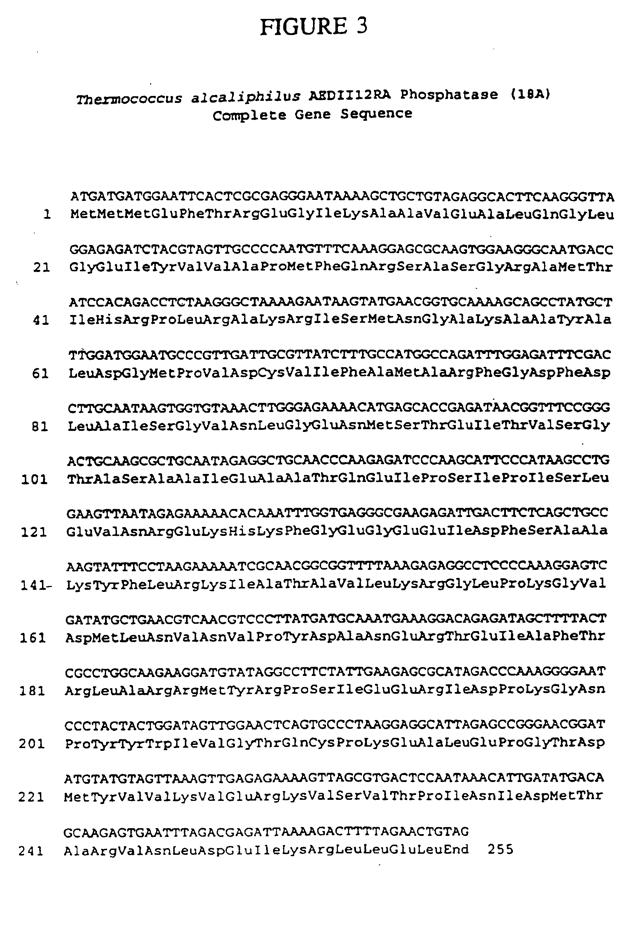

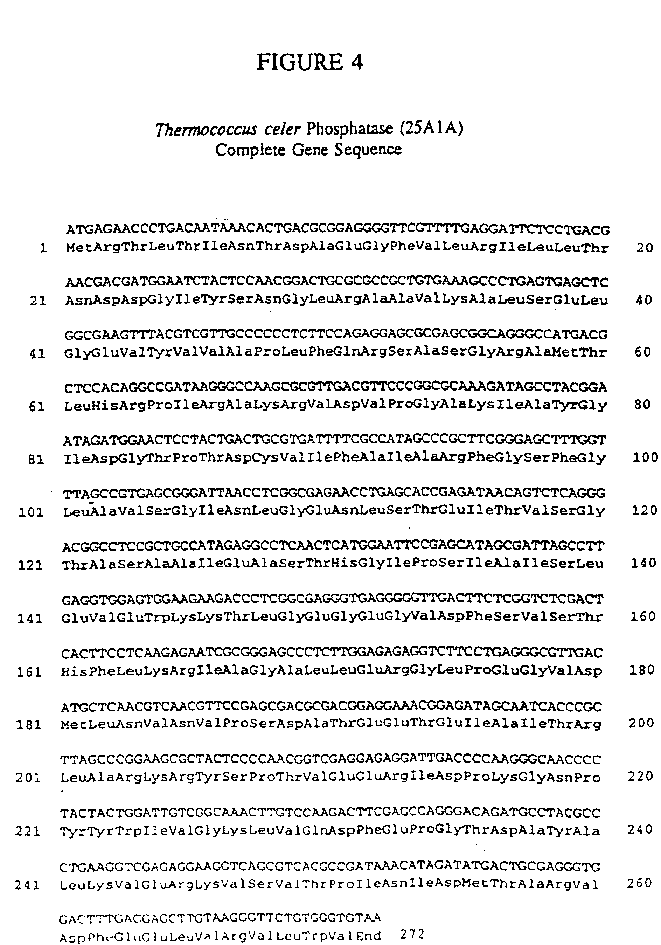

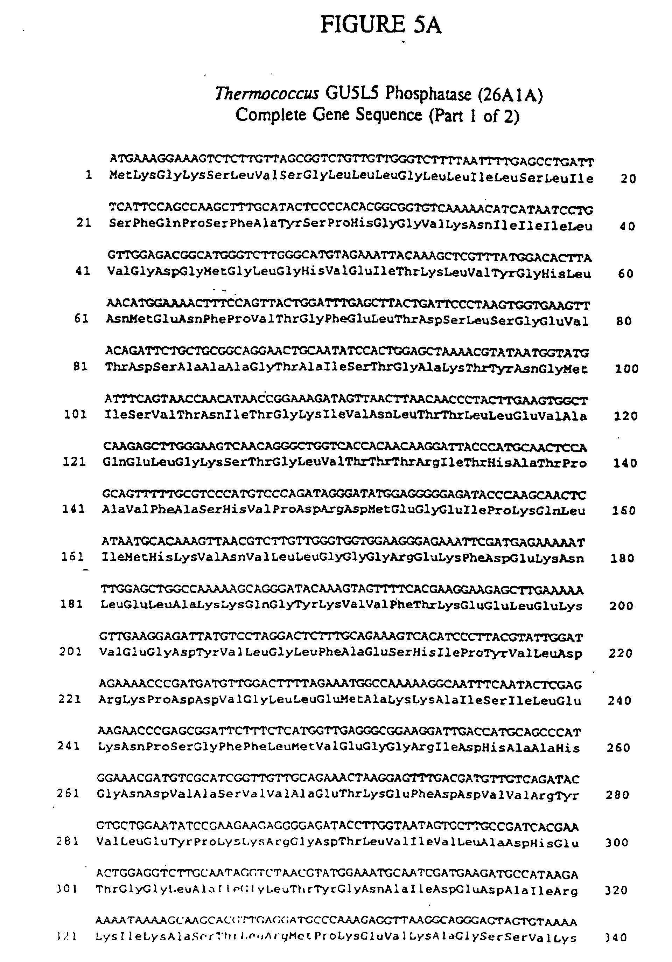

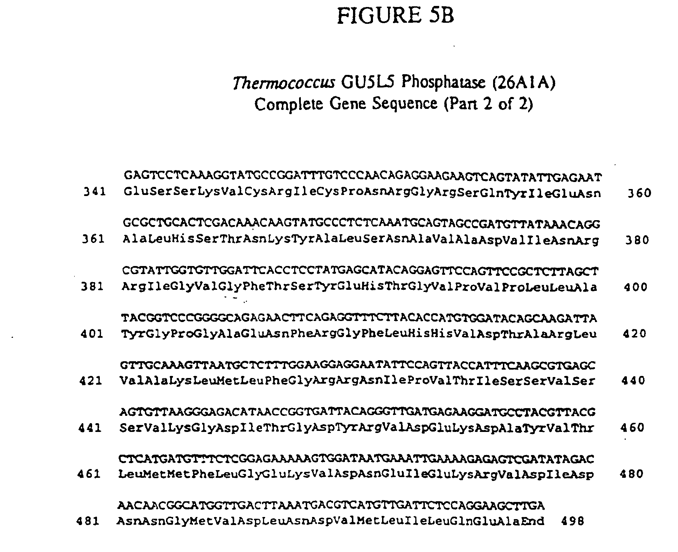

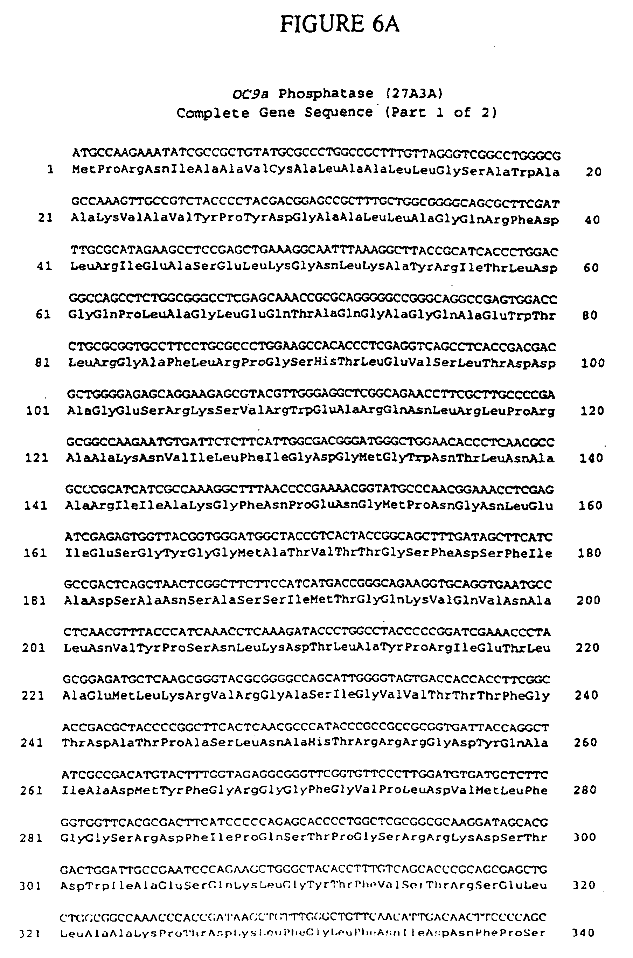

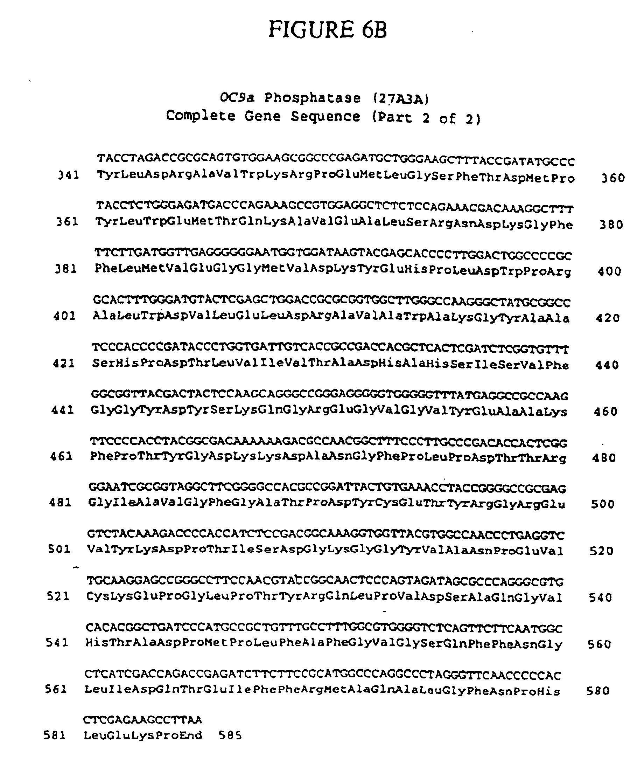

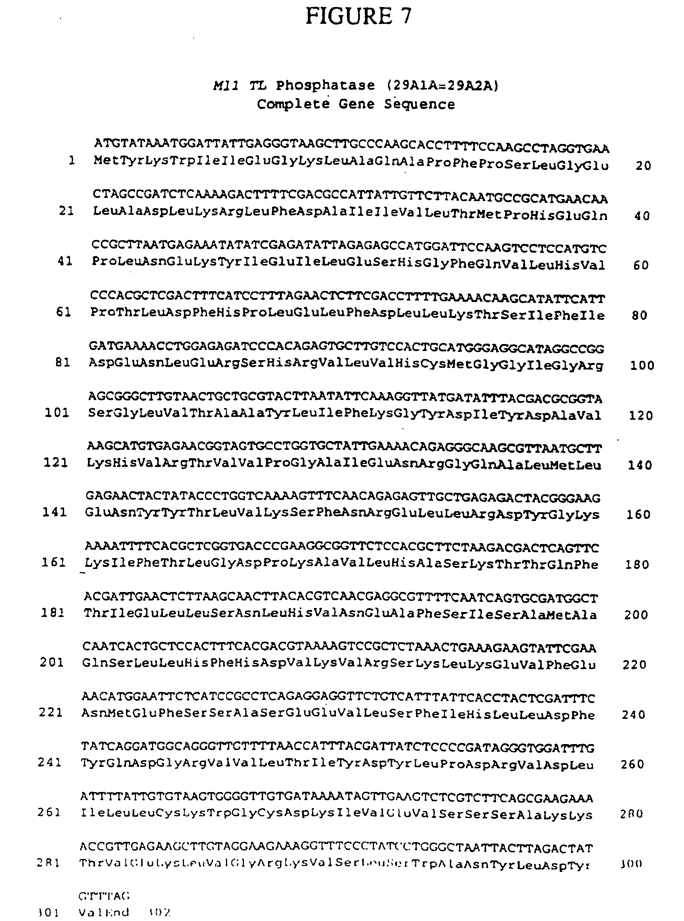

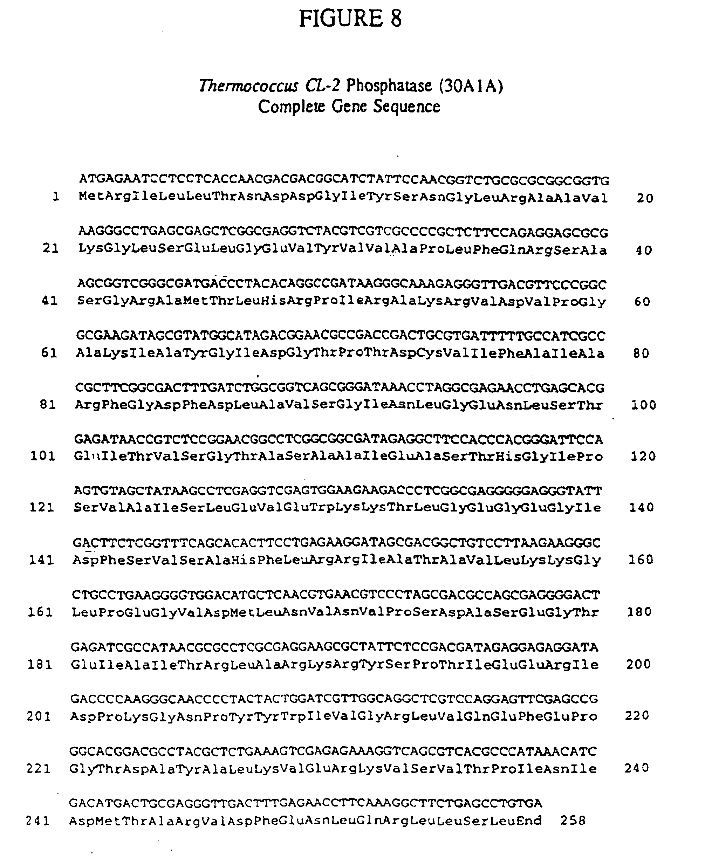

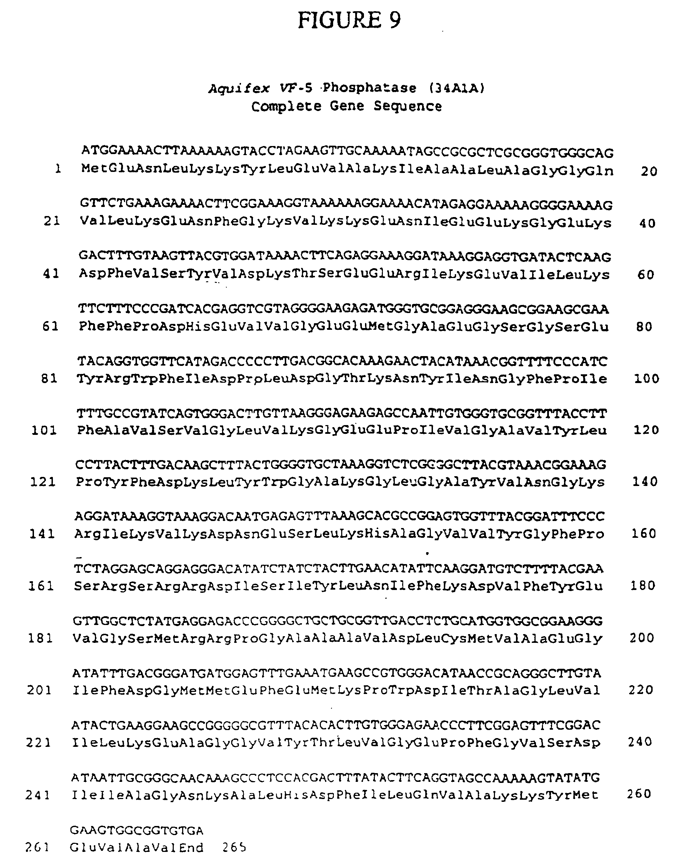

Accordingly, the polynucleotides and enzymes encoded thereby are identified by the organism from which they were isolated, and are sometimes hereinafter referred to as "KC4" (Figure 1 and SEQ ID NOS:19 and 28) , "Ko15" (Figure 2 and SEQ ID NOS:20 and 29), "AEDII12RA" (Figure 3 and SEQ ID NOS:21 and 30), "Celer" (Figure 4 and SEQ ID NOS:22 and 31), "GU5L5" (Figure 5 and SEQ ID NOS:23 and 32), "OC9a" (Figure 6 and SEQ ID NOS:24 and 33), "M11TL" (Figure 7 and SEQ ID NOS:25 and 34), "CL-2" (Figure 8 and SEQ ID NOS:26 and 35) and "VF-5" (Figure 9 and SEQ ID NOS:27 and 36).

The polynucleotides and polypeptides of the present invention show identity of the nucleotide and protein level to known genes and proteins encoded thereby as shown in Table 1.

**Table 1**

| Clone | Gene/Protein with Closest Homology | Protein Identity | Nucleic Acid Identity |
|---|---|---|---|
| *Ammonifex degensiii* KC4-3A1A | *Yarrowia lipolytica, Candida lipolytica*, acid phosphatase | 47% | 24% |
| *Ammonifex degensii* KC4-3A1A | *Saccharomyces cerevisiae*, hypothetical protein YBR094w | 54% | 26% |
| *Methanococcus igeneus* Ko15-9A1A | *Yarrowia lipolytica, Candida lipolytica*, acid phosphatase | 45% | 25 % |
| *Methanococcus igeneus* Ko15-9A1A | *Saccharomyces cerevisiae*, hypothetical protein YBR094w, hypothetical protein YBR0821 | 52% | 25% |
| *Thermococcus alcaliphilus* AEDII12RA-18A | No homology found | -- | -- |
| *Thermococus celer* 25A1A | No homology found | -- | -- |
| *Thermococcus* GU5L5-26A1A | *Bacillius subtilis*, alkaline phosphatase IV precursor, alkaline phosphomonoesterase, glycerophosphatase, and phosphomonoesterase | 58% | 38% |
| *Thermococcus* GU5L5-26A1A | *Bacillius subtills*, alkaline phosphatase III precursor | 58% | 37% |
| OC9a-27A3A | *Bombyx mori* (silkworm), alkaline phosphatase precursor | 54% | 33 % |
| OC9a - 27A3A | *Escherichia coli* C, alkaline phosphatase precursor | 53% | 34% |
| M11 TL - 29A1A | *Rhodobacrer capsulatus*, hypothetical protein B | 43% | 24% |
| *Thermococcus* C12-30A1A | *Yarrowia lipolytica, Candida lipolytica*, acid phosphatase | 49% | 27% |
| *Thermococcus* CL2-30A1A | *Saccharomyces cerevisiae*, hypothetical protein YBR094w hypothetical protein YBR0821 | 50% | 25% |
| *Aquifex* VF5-34A1A | *Escherichia coli*, suppressor protein suhB | 57% | 34% |

All of the clones identified in Table 1 encode polypeptides which have phosphatase activity.

One means for isolating the nucleic acid molecules encoding the enzymes of the present invention is to probe a gene library with a natural or artificially designed probe using art recognized procedures (see, for example: Current Protocols in Molecular Biology, Ausubel F.M. et al. (EDS.) Green Publishing Company Assoc. and John Wiley Interscience, New York, 1989, 1992). It is appreciated by one skilled in the art that the polynucleotides of SEQ ID NOS: 1-18, or fragments thereof (comprising at least 12 contiguous nucleotides), are particularly useful probes. Other particularly useful probes for this purpose are hybridizable fragments of the sequences of SEQ ID NOS: 19-27 (i.e., comprising at least 12 contiguous nucleotides).

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at (Tm less 10°C) for the oligonucleotide probe. The membrane is then exposed to autoradiographic film for detection of hybridization signals.

Stringent conditions means hybridization will occur only if there is at least 90% identity, preferably at least 95% identity and most preferably at least 97% identity between the sequences. Further, it is understood that a section of a 100 bps sequence that is 95 bps in length has 95% identity with the 1090 bps sequence from which it is obtained. See J. Sambrook *et al*., *Molecular Cloning, A Laboratory Manual, 2d Ed*., Cold Spring Harbor Laboratory (1989) which is hereby incorporated by reference in its entirety. Also, it is understood that a fragment of a 100 bps sequence that is 95 bps in length has 95% identity with the 100 bps sequence from which it is obtained.

As used herein, a first DNA (RNA) sequence is at least 70% and preferably at least 80% identical to another DNA (RNA) sequence if there is at least 70% and preferably at least a 80% or 90% identity, respectively, between the bases of the first sequence and the bases of the another sequence, when properly aligned with each other, for example when aligned by BLASTN.

The present invention relates to polynucleotides which differ from the reference polynucleotide such that the differences are silent, for example, the amino acid sequence encoded by the polynucleotides is the same. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

The polynucleotides of this invention were recovered from genomic gene libraries from Thermococcus. Gene libraries were generated from either of a Lambda ZAP I1 or a pBluscript] cloning vector (Stratagene Cloning Systems). Mass excisions were performed on these libraries to generate libraries in the pBluescript phagemid. Libraries were generated and excisions were performed according to the protocols/methods hereinafter described.

The polynucleotides of the present invention may be in the form of RNA or DNA which DNA includes cDNA, genomic DNA, and synthetic DNA. The DNA may be double-stranded or single-stranded, and if single stranded may be the coding strand or non-coding (anti-sense) strand. The coding sequences which encodes the mature enzymes may be identical to the coding sequences shown in Figures 1-9 (SEQ ID NOS: 21, 22, 23, 26) or may be a different coding sequence which coding sequence, as a result of the redundancy or degeneracy of the genetic code, encodes the same mature enzymes as the DNA of Figures 1-9 (SEQ ID NOS: 21, 22, 23, 26).

The polynucleotide which encodes for the mature enzyme of Figures 1-9 (SEQ ID NOS: 30, 31, 32, 35) may include, but is not limited to: only the coding sequence for the mature enzyme; the coding sequence for the mature enzyme and additional coding sequence such as a leader sequence or a proprotein sequence; the coding sequence for the mature enzyme (and optionally additional coding sequence) and non-coding sequence, such as introns or non-coding sequence 5' and/or 3' of the coding sequence for the mature enzyme.

Thus, the term "polynucleotide encoding an enzyme (protein)" encompasses a polynucleotide which includes only coding sequence for the enzyme as well as a polynucleotide which includes additional coding and/or non-coding sequence.

The present invention further relates to variants of the hereinabove described polynucleotides which encode for fragments, analogs and derivatives of the enzymes having the deduced amino acid sequences of Figures 1-9 (SEQ ID NOS: 30, 31, 32, 35). The variant of the polynucleotide may be a naturally occurring allelic variant of the polynucleotide or a non-naturally occurring variant of the polynucleotide.

The specification describes polynucl eot ides encoding the same mature enzymes as shown in Figures 1-9 (SEQ ID NOS: 19-27) as well as variants of such polynucleotides which variants encode for a fragment, derivative or analog of the enzymes of Figures 1-9 (SEQ ID NOS: 19-27). Such nucleotide variants include deletion variants, substitution variants and addition or insertion variants.

As hereinabove indicated, the polynucleotides may have a coding sequence which is a naturally occurring allelic variant of the coding sequences shown in Figures 1-9 (SEQ ID NOS: 19-27). As known in the art, an allelic variant is an alternate form of a polynucleotide sequence which may have a substitution, deletion or addition of one or more nucleotides, which does not substantially alter the function of the encoded enzyme. Also, using directed and other evolution strategies, one may make very minor changes in DNA sequence which can result in major changes in function.

, Fragments of the full length gene of the present invention may be used as hybridization probes for a cDNA or a genomic library to isolate the full length DNA and to isolate other DNAs which have a high sequence similarity to the gene or similar biological activity. Probes of this type preferably have at least 10, preferably at least 15, and even more preferably at least 30 bases and may contain, for example, at least 50 or more bases. In fact, probes of this type having at least up to 150 bases or greater may be preferably utilized. The probe may also be used to identify a DNA clone corresponding to a full length transcript and a genomic clone or clones that contain the complete gene including regulatory and promotor regions, exons and introns. An example of a screen comprises isolating the coding' region of the gene by using the known DNA sequence to synthesize an oligonucleotide probe. Labeled oligonucleotides having a sequence complementary or identical to that of the gene or portion of the gene sequences of the present invention are used to screen a library of genomic DNA to determine which members of the library the probe hybridizes to.

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

The specification further describes polynucleotides which hybridize to the hereinabove-described sequences if there is at least 70%, preferably at least 90%, and more preferably at least 95% identity between the sequences. (As indicated above, 70% identity would include within such definition a 70 bps fragment taken from a 100 bp polynucleotide, for example.) The present invention also relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides encode enzymes which either retain substantially the same biological function or activity as the mature enzyme encoded by the DNA of Figures 1-9 (SEQ ID NOS: 19-27). In referring to identity in the case of hybridization, as known in the art, such identity refers to the complementarity of two polynucleotide segments.

Alternatively, the polynucleotide may have at least 15 bases, preferably at least 30 bases, and more preferably at least 50 bases which hybridize to any part of a polynucleotide of the present invention and which has an identity thereto, as hereinabove described, and which may or may not retain activity. For example, such polynucleotides may be employed as probes for the polynucleotides of SEQ ID NOS: 19-27, for example, for recovery of the polynucleotide or as a diagnostic probe or as a PCR primer.

Thus, the specification describes polynucleotides having at least a 70% identity, preferably at least 90% identity and more. preferably at least a 95% identity to a polynucleotide which encodes the enzymes of SEQ ID NOS: 28-36 as well as fragments thereof, which fragments have at least 15 bases, preferably at least 30 bases, more preferably at least 50 bases and most preferably fragments having up to at least 150 bases or greater, which fragments are at least 90% identical, preferably at least 95% identical and most preferably at least 97% identical to any portion of a polynucleotide of the present invention.

The present invention relates to enzymes which have the deduced amino acid sequences of Figures 1-9 (SEQ ID NOS: 30, 31, 32, 35) as well as fragments, analogs and derivatives of such enzyme.

The terms "fragment," "derivative" and "analog" when referring to the enzymes of Figures 1-9 (SEQ ID NOS. 30, 31, 32, 35) means enzymes which retain essentially the same biological function or activity as such enzymes. Thus, an analog includes a proprotein which can be activated by cleavage of the proprotein portion to produce an active mature enzyme.

The enzymes of the present invention may be a recombinant enzyme, a natural enzyme or a synthetic enzyme, preferably a recombinant enzyme.

The fragment, derivative or analog of the enzymes of Figures 1-9 (SEQ ID NOS.28-36) may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature enzyme is fused with another compound, such as a compound to increase the half-life of the enzyme (for example, polyethylene glycol), or (iv) one in which the additional amino acids are fused to the mature enzyme, such as a leader or secretory sequence or a sequence which is employed for purification of the mature enzyme or a proprotein sequence. Such fragments, derivatives and analogs are deemed to be within the scope of those skilled in the art from the teachings herein.

The enzymes and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or enzyme present in a living animal is not isolated, but the same polynucleotide or enzyme, separated from some or all of the coexisting materials in the natural system, is isolated. Such polynucleotides could be part of a vector and/or such polynucleotides or enzymes could be part of a composition, and still be isolated in that such vector or composition is not part of its natural environment.

The enzymes described in the specification include the enzymes of SEQ ID NOS: 28-36 (in particular the mature enzyme) as well as enzymes which have at least 70% similarity (preferably at least 70% identity) to the enzymes of SEQ ID NOS: 28-36 and more preferably at least 90% similarity (more preferably at least 90% identity) to the enzymes of SEQ ID NOS: 28-36 and still more preferably at least 95% similarity (still more preferably at least 95% identity) to the enzymes of SEQ ID NOS: 28-36 and also include portions of such enzymes with such portion of the enzyme generally containing at least 30 amino acids and more preferably at least 50 amino acids and most preferably at least up to 150 amino acids.

As known in the art "similarity" between two enzymes is determined by comparing the amino acid sequence and its conserved amino acid substitutes of one enzyme to the sequence of a second enzyme. The definition' of 70% similarity would include a 70 amino acid sequence fragment of a 100 amino acid sequence, for example, or a 70 amino acid sequence obtained by sequentially or randomly deleting 30 amino acids from the 100 amino acid sequence.

A variant, *i.e.* a "fragment", "analog" or "derivative" polypeptide, and reference polypeptide may differ in amino acid sequence by one or more substitutions, additions, deletions, fusions and truncations, which may be present in any combination.

Among preferred variants are those that vary from a reference by conservative amino acid substitutions. Such substitutions are those that substitute a given amino acid in a polypeptide by another amino acid of like characteristics. Typically seen as conservative substitutions are the replacements, one for another, among the aliphatic amino acids Ala, Val, Leu and Ile; interchange of the hydroxyl residues Ser and Thr, exchange of the acidic residues Asp and Glu, substitution between the amide residues Asn and Gln, exchange of the basic residues Lys and Arg and replacements among the aromatic residues Phe, Tyr.

Most highly preferred are variants which retain the same biological function and activity as the reference polypeptide from which it varies.

Fragments or portions of the enzymes of the present invention may be employed for producing the corresponding full-length enzyme by peptide synthesis; therefore, the fragments may be employed as intermediates for producing the full-length enzymes. Fragments or portions of the polynucleotides of the present invention may be used to synthesize full-length polynucleotides of the present invention.

The present invention also relates to vectors which include polynucleotides of the present invention, host cells which are genetically engineered with vectors of the invention and the production of enzymes of the invention by recombinant techniques.

Host cells are genetically engineered (transduced or transformed or transfected) with the vectors of this invention which may be, for example, a cloning vector such as an expression vector. The vector may be, for example, in the form of a plasmid, a phage, *etc*. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing enzymes by recombinant techniques. Thus, for example, the polynucleotide may be included in any one of a variety of expression vectors for expressing an enzyme. Such vectors include chromosomal, nonchromosomal and synthetic DNA sequences, *e.g*., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the *E. coli. lac or trp,* the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in *E. coli*.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as *E. coli*, *Streptomyces, Bacillus subtilis*; fungal cells, such as yeast; insect cells such as *Drosophila S2* and *Spodoptera Sf9;* animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQE70, pQE60, pQE-9 (Qiagen), pBluescript II KS, ptrc99a, pKK223-3, pDR540, pRIT2T-(Pharmacia); Eukaryotic: pXT1, pSG5 (Stratagene) pSVK3, pBPV, pMSG, pSVL SV40 (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., *Basic Methods in Molecular Biology*, (1986)).

The constructs in host cells can be used in a conventional manner to produce the gene product encoded by the recombinant sequence. Alternatively, the enzymes of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook *et al., Molecular Cloning: A Laboratory Manual, Second Edition,* Cold Spring Harbor, N.Y., (1989), the disclosure of which is hereby incorporated by reference.

Transcription of the DNA encoding the enzymes of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, *e.g*., the ampicillin resistance gene of *E. coli* and *S. cerevisiae* TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated enzyme. Optionally, the heterologous sequence can encode a fusion enzyme including an N-terminal identification peptide imparting desired characteristics, *e.g*., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include *E. coli*, *Bacillus subtilis, Salmonella typhimurium* and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and pGEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (*e.g*., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well known to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The enzyme can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The enzymes of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the enzymes of the present invention may be glycosylated' or may be non-glycosylated. Enzymes of the invention may or may not also include an initial methionine amino acid residue.

Phosphatases are a group of key enzymes in the removal of phosphate groups from organophosphate ester compounds. There are numerous phosphatases, including alkaline phosphatases, phosphodiesterases and phytases.

The general application and definitions of such compounds are discussed above under the background of the invention section.

The present invention provides novel phosphatase enzymes having enhanced thermostability. Such phosphatases are beneficial in enzyme labeling processes and in certain recombinant DNA techniques, such as in the dephosphorylation of vector DNA prior to insert DNA ligation. The recombinant phosphatase enzymes provide the proteins in a format amenable to efficient production of pure enzyme, which can be utilized in a variety of applications as described herein.

The specification also describes antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate-antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, Nature, 256:495-497, 1975), the trioma technique, the human B-cell hybridoma technique (Kozbor *et al*., Immunology Today 4:72, 1983)., and the EBV-hybridoma technique to produce human monoclonal antibodies (Cole *et al*., in *Monoclonal Antibodies and Cancer Therapy,* Alan R. Liss, Inc., pp. 77-96, 1985).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

The specification also describes antibodies generated against an enzyme of the present invention may be used in screening for similar enzymes from other organisms and samples. Such screening techniques are known in the art, for example, one such screening assay is described in Sambrook and Maniatis, Molecular Cloning: A Laboratory Manual (2d Ed.), vol. 2:Section 8.49, Cold Spring Harbor Laboratory, 1989.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case "p" preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel *et al*., *Nucleic Acids Res*., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., *et al*., *Id*., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in Sambrook and Maniatis, Molecular Cloning: A Laboratory Manual, Cold Spring Harbor Laboratory, 1989.

One means for isolating the nucleic acid molecules encoding the enzymes of the present invention is to probe a gene library with a natural or artificially designed probe using art recognized procedures (see, for example: Current Protocols in Molecular Biology, Ausubel F.M. *et al*. (EDS.) Green Publishing Company Assoc. and John Wiley Interscience, New York, 1989, 1992). It is appreciated to one skilled in the art that the polynucleotides of SEQ ID NOS:1-16, or fragments thereof (comprising at least 10 or 12 contiguous nucleotides), are particularly useful probes. Other particularly useful probes for this purpose are fragments hybridizable fragments to the sequences of SEQ ID NOS:19-27 (*i.e.*, comprising at least 10 or 12 contiguous nucleotides).

It is also appreciated that such probes can be and are preferably labeled with an analytically detectable reagent to facilitate identification of the probe. Useful reagents include but are not limited to radioactivity, fluorescent dyes or enzymes capable of catalyzing the formation of a detectable product. The probes are thus useful to isolate complementary copies of DNA from other sources or to screen such sources for related sequences.

With respect to nucleic acid sequences which hybridize to specific nucleic acid sequences disclosed herein, hybridization may be carried out under conditions of reduced stringency, medium stringency or even stringent conditions. As an example of oligonucleotide hybridization, a polymer membrane containing immobilized denatured nucleic acids is first prehybridized for 30 minutes at 45°C in a solution consisting of 0.9 M NaCl, 50 mM NaH₂PO₄, pH 7.0, 5.0 mM Na₂EDTA, 0.5% SDS, 10X Denhardt's, and 0.5 mg/mL polyriboadenylic acid. Approximately 2 X 10⁷ cpm (specific activity 4-9 X 10⁸ cpm/ug) of ³²P end-labeled oligonucleotide probe are then added to the solution. After 12-16 hours of incubation, the membrane is washed for 30 minutes at room temperature in 1X SET (150 mM NaCl, 20 mM Tris hydrochloride, pH 7.8, 1 mM Na₂EDTA) containing 0.5% SDS, followed by a 30 minute wash in fresh 1X SET at Tm -10°C for the oligonucleotide probe. The membrane is then exposed to autoradiographic film for detection of hybridization signals.

Stringent conditions means hybridization will occur only if there is at least 90% identity, preferably 95% identity and most preferably at least 97% identity between the sequences. See J. Sambrook et al., Molecular Cloning, A Laboratory Manual (2d Ed. 1989) (Cold Spring Harbor Laboratory) which is hereby incorporated by reference in its entirety.

"Identity" as the term is used herein, refers to a polynucleotide sequence which comprises a percentage of the same bases as a reference polynucleotide (SEQ ID NOS:1-16). For example, a polynucleotide which is at least 90% identical to a reference polynucleotide, has polynucleotide bases which are identical in 90% of the bases which make up the reference polynucleotide and may have different bases in 10% of the bases which comprise that polynucleotide sequence.

The present invention relates to polynucleotides which differ from the reference polynucleotide such that the differences are silent changes, for example, the amino acid sequence encoded by both polynucleotides is the same. The present invention also relates to nucleotide changes which result in amino acid substitutions, additions, deletions, fusions and truncations in the polypeptide encoded by the reference polynucleotide. In a preferred aspect of the invention these polypeptides retain the same biological action as the polypeptide encoded by the reference polynucleotide.

The polynucleotides of this invention were recovered from genomic gene libraries from the organisms listed in Table 1. Gene libraries were generated in the Lambda ZAP II cloning vector (Stratagene Cloning Systems). Mass excisions were performed on these libraries to generate libraries in the pBluescript phagemid. Libraries were generated and excisions were performed according to the protocols/methods hereinafter described.

The excision libraries were introduced into the E. coli strain BW14893 F'kan1A. Expression clones were then identified using a high temperature filter assay using phosphatase buffer containing 1 mg/ml BCIP (5-Bromo-4-chloro-3-indolyl phosphate). Expression clones encoding BCIPases were identified and repurified from the following organisms: *Ammonifex degensii* KC4, *Methanococcus igneus* KoL5, *Thermococcus alcaliphilus* AED112RA, *Thermococcus celer, Thermococcus* GU5L5, *OC9a,* M11TL, *Thermococcus* CL-2 and *Aquifex* VF-5.

Expression clones were identified by use of a high temperature filter assay with either acid phosphatase buffer or alkaline phosphatase buffer containing BCIP. Metcalf, *et al*., Evidence for two phosphonate degradative pathways in Enterobacter Aerogenes, J. Bacteriol., 174:2501-2510 (1992)).

BCIPase activity was tested as follows: An excision library was introduced into the *E. Coli* strain BW14893 F'kan, a pho pnh lac strain. After growth on 100 mm LB plates containing 100 µg/ml ampicillin, 80 µg/ml methicillin and 1mM IPTG, colony lifts were performed using Millipore HATF membrane filters. The colonies transferred to the filters were lysed with chloroform vapor in 150 mm glass petri dishes. The filters were transferred to 100 mm glass petri dishes containing a piece of Whatman 3MM filter paper saturated with either acid phosphatase buffer (see recipe below) or alkaline phosphatase buffer (see recipe below) containing no BCIP. The dish was placed in the oven at 80-85°C for 30-45 minutes to heat inactivate endogenous *E. coli* phosphatases. The filter bearing lysed colonies were then transferred to a 100 mm glass petri dish containing 3MM paper saturated with either acid phosphatase buffer or alkaline phosphatase buffer containing 1 mg/ml BCIP. The dish was placed in the oven at 80-85°C.

Alkaline Phosphatase Buffer (referenced in Sambrook, J. *et al*. (1989) *Molecular Cloning, A Laboratory Manual,* p. 1874) includes 100 mM NaCl, 5 mM MgCl₂ and 100 mM Tris-HCl (pH 9.5). Clones expressing phosphatase activity (when the alkaline phosphatase buffer was used) were derived from libraries derived from the organism identified above.

Acid Phosphatase Buffer includes 100 mM NaCl, 5 mM MgCl₂ and 100 mM Tris-HCL (pH 6.8). Clones expressing phosphatase activity (when the acid phosphatase buffer was used) were derived from the library derived from M11TL.

'Positives' were observed as blue spots on the filter membranes. The following filter rescue technique was used to retrieve plasmid from lysed positive colony.

Filter Rescue Technique: A pasteur pipette (or glass capillary tube) was used to core blue spots on the filter membrane. The small filter disk was placed in an Eppendorf tube containing 20 ul of deionized water. The Eppendorf tube was incubated at 75°C for 5 minutes followed by vortexing to elute plasmid DNA off the filter. Plasmid DNA containing DNA inserts from *Thermococcus alcaliphilus* AEDII12RA was used to transform electrocompetent *E. coli* DH10B cells. Electrocompetent BW14893 F'kanlA *E. coli* cells were used for transformation of plasmid DNA containing inserts from *Ammonifex degensii* KC4, *Methanococcus igneus* KOL5, and *Thermococcus* GU5L5. The filter-lift assay was repeated on transformation plates to identify 'positives.' The transformation plates were returned to 37°C incubator to regenerate colonies. 3 ml of LBamp liquid was inoculated with repurified positives and incubated at 37°C overnight. Plasmid DNA was isolated from these cultures and plasmid insert were sequenced.

In some instances where the plates used for the initial colony lifts contained non-confluent colonies, a specific colony corresponding to a blue spot on the filter could be identified on a regenerated plate and repurified directly, instead of using the filter rescue technique. This "repurification" protocol was used for plasmid DNA containing inserts from the following: *Ammonifex degensii* KC4, *Thermococcus celer*, M11TL, and *Aquifex* VF-5.

The filter rescue technique was used for DNA from the following organisms: *Ammonifex degensii* KC4, *Methanococcus igneus* KOL5, *Thermococcus alcaliphilus* AED1112RA, *Thermococcus* CL-2, and OC9a.

Phosphatases are a group of key enzymes that remove phosphate groups from organophosphate ester compounds. The most important phosphatases for commercial purposes are alkaline phosphatases, phosphodiesterases, and phytases.

Alkaline phosphatases have several commercial applications, including their use in analytical applications as an enzyme label in ELISA immunoassays and enzyme-linked gene probes, and their use in research applications for removing 5' phosphates in polynucleotides prior to end-labeling and for dephosphorylating vectors prior to insert ligation (see also Current Protocols in Molecular Biology, (John Wiley & Sons) (1995), chapter 3, section 10).

Alkaline phosphatase hydrolyzes monophosphate esters, releasing inorganic phosphate and. the cognate alcohol compound. It is non-specific with respect to the alcohol moiety, a feature which accounts for the many uses of this enzyme. The enzyme has a pH optimum between 9 and 10, however, it can also work at neutral pH. (From a study of the enzyme industry conducted by Business Communications, Co., Inc., 25 Van Zant Street, Norwalk, CT 06855, 1995.)

Two sources of alkaline phosphatase dominate and compete in the market: animal, from bovine and calf intestinal mucosa, and bacterial, from *E. coli.* Due to the high turnover number of calf intestinal phosphatase, it is often selected as the label in many enzyme immunoassays. The usefulness of calf alkaline phosphatase is limited by its inherently low thermal stability, which is even-further compromised during the chemical preparation of enzyme: antibody conjugates. Bacterial alkaline phosphatase could be an attractive alternative to calf alkaline phosphatase due to bacterial alkaline phosphatase's extreme thermotolerance at temperatures as high as 95°C. (Tomazic-Allen S.J., Recombinant bacterial alkaline phosphatase as an immunodiagnostic enzyme, *Annales de Biologie Clinique*, 1991, 49(5):287-90).

The specification also describes antibodies generated against the enzymes corresponding to a sequence of the present invention can be obtained by direct injection of the enzymes into an animal or by administering the enzymes to an animal, preferably a nonhuman. The antibody so obtained will then bind the enzymes itself. In this manner, even a sequence encoding only a fragment of the enzymes can be used to generate antibodies binding the whole native enzymes. Such antibodies can then be used to isolate the enzyme from cells expressing that enzyme.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic enzyme products of this invention. Also, transgenic mice may be used to express humanized antibodies to immunogenic enzyme products of this invention.

The specification describes antibodies which may be employed as a probe to screen a library to-identify the above-described activities or cross-reactive activities in gene libraries generated from the organisms described above or other organisms.

The following examples cover also embodiments which are not part of the invention.

### Example 1

### Bacterial Expression and Purification of Alkaline Phosphatase Enzymes

DNA encoding the enzymes described in the specification, SEQ ID NOS:1 through 16, were initially amplified from a pBluescript vector containing the DNA by the PCR technique using the primers noted herein. The amplified sequences were then inserted into the respective pQE vector listed beneath the primer sequences, and the enzyme was expressed according to the protocols set forth herein. The 5' and 3' oligonucleotide primer sequences used for subcloning and vectors for the respective genes are as follows:

The restriction enzyme sites indicated correspond to the restriction enzyme sites on the bacterial expression vector indicated for the respective gene (Qiagen, Inc. Chatsworth, CA). The pQE vector encodes antibiotic resistance (Amp^{r}), a bacterial origin of replication (ori), an IPTG-regulatable promoter operator (P/O), a ribosome binding site (RBS), a 6-His tag and restriction enzyme sites.

The pQE vector was digested with the restriction enzymes indicated. The amplified sequences were ligated into the respective pQE vector and inserted in frame with the sequence encoding for the RBS. The native stop codon was incorporated so the genes were not fused to the His tag of the vector. The ligation mixture was then used to transform the E. coli strain M15/pREP4 (Qiagen, Inc.) by electroporation. M15/pREP4 contains multiple copies of the plasmid pREP4, which expresses the lacI repressor and also confers kanamycin resistance (Kan^{r}). Transformants were identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture was used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation.

The primer sequences set out above may also be employed to isolate the target gene from the deposited material by hybridization techniques described above.

### Example 2

### Isolation of A Selected Clone From the Deposited Genomic Clones

A clone is isolated directly by screening the deposited material using the oligonucleotide primers set forth in Example 1 for the particular gene desired to be isolated. The specific oligonucleotides are synthesized using an Applied Biosystems DNA synthesizer.

The two oligonucleotide primers corresponding to the gene of interest are used to amplify the gene from the deposited material. A polymerase chain reaction is carried out in 25 µl of reaction mixture with 0.1 ug of the DNA of the gene of interest. The reaction mixture is 1.5-5 mM MgCl₂, 0.01% (w/v) gelatin, 20 µM each of dATP, dCTP, dGTP,-dTTP, 25 pmol of each primer and 1.25 Unit of Taq polymerase. Thirty cycles of PCR (denaturation at 94°C for 1 min; annealing at 55°C for 1 min; elongation at 72°C for 1 min) are performed with the Perkin-Elmer Cetus 9600 thermal cycler. The amplified product is analyzed by agarose gel electrophoresis and the DNA band with expected molecular weight is excised and purified. The PCR product is verified to be the gene of interest by subcloning and sequencing the DNA product. The ends of the newly purified genes are nucleotide sequenced to identify full length sequences. Complete sequencing of full length genes is then performed by Exonuclease III digestion or primer walking.

Numercus modifications and variations of the present invention are possible in light of the above teachings and, therefore, within the scope of the appended claims, the invention may be practiced otherwise than as particularly described.

## Claims

1. A polynucleotide selected from the group consisting of:
(a) a nucleic acid sequence encoding a polypeptide as set forth in SEQ ID NO:30, SEQ ID NO:31, SEQ ID NO:32, SEQ ID NO:35, or SEQ ID NO:46;
(b) a nucleic acid sequence as set forth in SEQ ID NO:21, SEQ ID NO:22, SEQ ID NO:23, SEQ ID NO:26, SEQ ID NO:38 or SEQ ID NO:45;
(c) the nucleic acid sequence of (a) or (b), wherein T can also be a U;
(d) a nucleic acid sequence which is at least 15, 30 or 50 consecutive bases in length of a sequence as set forth in SEQ ID NO:21, SEQ ID NO:22, SEQ ID NO:23, SEQ ID NO:26, SEQ ID NO:38 or SEQ ID NO:45;
(e) a nucleic acid probe which is at least 15, 30 or 50 consecutive bases in length that will selectively hybridize to the nucleic acid sequence of
(a) to (d);
(f) a nucleic acid sequence encoding an enzyme having alkaline phosphatase activity having at least 70%, 90%, 95% or 97% sequence identity to SEQ ID NO:21, SEQ ID NO:22, SEQ ID NO:23, SEQ ID NO:26, SEQ ID NO:38 or SEQ ID NO:45; and
(g) a nucleic acid sequence complementary to the nucleic acid sequence of (a) to (f);
optionally including additional coding and/or non-coding sequence.

2. The polynucleotide of claim 1, wherein the polynucleotide is DNA.

3. The polynucleotide of claim 1, wherein the polynucleotide is RNA.

4. A polynucleotide comprising a member selected from the group consisting of:
(a) a polynucleotide encoding an enzyme having alkaline phosphatase activity and having at least 70% identity to a polynucleotide encoding an enzyme encoded by the DNA contained in ATCC deposit No. 97536, wherein said enzyme is selected from the group consisting of Thermococcus AEDII12RA, Thermococcus celer, Thermococcus CL-2 and Thermococcus GU5L5;
(b) a polynucleotide complementary to the polynucleotide of (a); and
(c) a polynucleotide comprising at least 15 bases of the polynucleotide of (a) and (b).

5. A vector comprising the polynucleotide of any one of claims 1 to 4.

6. A host cell comprising the vector of claim 5.

7. A process for producing a polypeptide comprising: expressing from the host cell of claim 6 a polypeptide encoded by said DNA and isolating the polypeptide.

8. A process for producing a recombinant cell comprising: transforming or transfecting the cell with the vector of claim 5 such that the cell expresses the polypeptide encoded by the DNA contained in the vector.

9. An enzyme of which at least a portion of which is coded for by a polynucleotide selected from the group consisting of:
(a) an amino acid sequence as set forth in SEQ ID NO:30, SEQ ID NO:31, SEQ ID NO:32, SEQ ID NO:35, or SEQ ID NO:46;
(b) an amino acid sequence encoded by the polynucleotide of claim 1 or claim 4;
(c) an amino acid sequence at least 30 consecutive amino acids in length of the sequence as set forth in SEQ ID NO:30, SEQ ID NO:31, SEQ ID NO:32, SEQ ID NO:35, or SEQ ID NO:46 or a polypeptide encoded by the polynucleotide of claim 1;
(d) an amino acid sequence having an alkaline phosphatase activity and having at least 70%, 90%, 95% or 97% sequence identity to SEQ ID NO:30, SEQ ID NO:31, SEQ ID NO:32, SEQ ID NO:35, or SEQ ID NO:46 or a polypeptide encoded by the polynucleotide of claim 1.

10. A method for hydrolyzing phosphate bonds comprising: administering an effective amount of an enzyme selected from the group consisting of an enzyme having the amino acid sequence selected from the group of amino acid sequences set forth in SEQ ID NOS: 30, 31, 32, 35 or 46, an enzyme of claim 9 or an enzyme encoded by a polynucleotide of any one of claims 1 to 4.

11. A thermostable alkaline phosphatase having a sequence as set forth in claim 9 or encoded by the polynucleotide of claim 1(a), (b), (c) or (f).

12. An antibody generated against the enzyme of claim 9 or generated against a fragment of the enzyme which binds to whole native enzymes.

## Patentansprüche

1. Polynucleotid ausgewählt aus der Gruppe bestehend aus:
(a) einer Nucleinsäuresequenz, die ein Polypeptid codiert wie in SEQ ID Nr.: 30, SEQ ID Nr.: 31, SEQ ID Nr.: 32, SEQ ID Nr.: 35 oder SEQ ID Nr.: 46 gezeigt;
(b) einer Nucleinsäuresequenz wie in SEQ ID Nr.: 21, SEQ ID Nr.: 22; SEQ ID Nr.: 23, SEQ ID Nr.: 26, SEQ ID Nr.: 38 oder SEQ ID Nr.: 45 gezeigt;
(c) der Nucleinsäuresequenz von (a) oder (b), wobei T auch U sein kann;
(d) einer Nucleinsäuresequenz, die mindestens 15, 30 oder 50 aufeinanderfolgende Basen einer Sequenz wie in SEQ ID Nr.: 21, SEQ ID Nr.: 22, SEQ ID Nr.: 23, SEQ ID Nr.: 26, SEQ ID Nr.: 38 oder SEQ ID Nr.: 45 gezeigt, lang ist;
(e) einer Nucleinsäuresonde, die mindestens 15, 30 oder 50 aufeinanderfolgende Basen lang ist, welche spezifisch an die Nucleineinsäuresequenz von (a) bis (d) hybridisiert;
(f) einer Nucleinsäuresequenz, die ein Enzym mit alkalischer Phosphatase-Aktivität codiert, das mindestens 70%, 90%, 95% oder 97% Sequenzidentität zu SEQ ID Nr.: 21, SEQ ID Nr.: 22, SEQ ID Nr.: 23, SEQ ID Nr.: 26, SEQ ID Nr.: 38 oder SEQ ID Nr.: 45 aufweist; und
(g) einer Nucleinsäuresequenz, die zu der Nucleinsäuresequenz von (a) bis (f) komplementär ist;
gegebenenfalls einschließlich zusätzlicher codierender und/oder nichtcodierender Sequenz.

2. Polynucleotid nach Anspruch 1, wobei das Polynulceotid DNA ist.

3. Polynucleotid nach Anspruch 1, wobei das Polynucleotid RNA ist.

4. Polynucleotid umfassend ein Mitglied ausgewählt aus der Gruppe bestehend aus:
(a) einem Polynucleotid, das ein Enzym mit alkalischer Phosphatase-Aktivität codiert und mindestens 70% Identität zu einem Polynucleotid aufweist, das ein Enzym codiert, welches von der in ATCC-Hinterlegungsnummer Nr. 97536 enthaltenen DNA codiert wird, wobei das Enzym ausgewählt ist aus der Gruppe bestehend aus Thermococcus AEDII12RA, Thermococcus celer, Thermococcus CL-2 and Thermococcus GU5L5;
(b) einem Polynucleotid, das komplementär zu dem Polynulceotid von (a) ist;
(c) einem Polynucleotid, das mindestens 15 Basen des Polynucleotids von (a) und (b) umfasst.

5. Vektor, umfassend das Polynucleotid nach einem der Ansprüche 1 bis 4.

6. Wirtszelle, umfassend den Vektor nach Anspruch 5.

7. Verfahren zur Herstellung eines Polynucleotids umfassend: Exprimieren eines Polypeptids, das von der DNA codiert wird, von der Wirtszelle nach Anspruch 6 und Isolieren des Polypeptids.

8. Verfahren zur Herstellung einer rekombinanten Zelle umfassend: Transformieren oder Transfizieren der Zelle mit dem Vektor nach Anspruch 5, so dass die Zelle das Polypeptid exprimiert, das von der DNA codiert wird, die in dem Vektor enthalten ist.

9. Enzym, von dem zumindest ein Teil von einem Polynucleotid codiert wird, ausgewählt aus der Gruppe bestehend aus:
(a) einer Aminosäuresequenz wie in SEQ ID Nr.: 30, SEQ ID Nr.: 31, SEQ ID Nr.: 32, SEQ ID Nr.: 35 oder SEQ ID Nr.: 46 gezeigt;
(b) einer Aminosäuresequenz, die von dem Polynucleotid nach Anspruch 1 oder Anspruch 4 codiert wird;
(c) einer Aminosäuresequenz, die mindestens 30 aufeinanderfolgende Aminosäuren einer Sequenz wie in SEQ ID Nr.: 30, SEQ ID Nr.: 31, SEQ ID Nr.: 32, SEQ ID Nr.: 35 oder SEQ ID Nr.: 46 gezeigt, lang ist oder einem Polypeptid, das von dem Polynucleotid von Anspruch 1 codiert wird;
(d) einer Aminosäuresequenz mit alkalischer Phosphatase-Aktivität, die mindestens 70%, 90%, 95% oder 97% Sequenzidentität zu SEQ ID Nr.: 30, SEQ ID Nr.: 31, SEQ ID Nr.: 32, SEQ ID Nr.: 35 oder SEQ ID Nr.: 46 aufweist oder einem Polypeptid, das von dem Polynucleotid von Anspruch 1 codiert wird.

10. Verfahren zum Hydrolysieren von Phosphatbindungen umfassend: Zugeben einer wirksamen Menge eines Enzyms, ausgewählt aus der Gruppe bestehend aus einem Enzym mit der Aminosäuresequenz, ausgewählt aus der Gruppe von Aminosäuresequenzen wie in SEQ ID Nr.: 30, 31, 32, 35 oder 46 gezeigt, einem Enzym nach Anspruch 9 oder einem Enzym, das von dem Polynucleotid nach einem der Ansprüche 1 bis 4 codiert wird.

11. Thermostabile alkalische Phosphatase mit der Sequenz wie in Anspruch 9 gezeigt oder codiert von dem Polynucleotid nach Anspruch 1(a), (b), (c) oder (f).

12. Antikörper, erzeugt gegen das Enzym nach Anspruch 9 oder erzeugt gegen ein Fragment des Enzyms, der ganze native Enzyme bindet.

## Revendications

1. Polynucléotide sélectionné parmi le groupe constitué de :
(a) une séquence d'acide nucléique codant pour un polypeptide tel que présenté dans SEQ ID NO :30, SEQ ID NO :31, SEQ ID NO :32, SEQ ID NO :35 ou SEQ ID NO :46 ;
(b) une séquence d'acide nucléique telle que présentée dans SEQ ID NO :21, SEQ ID NO :22, SEQ ID NO :23, SEQ ID NO :26, SEQ ID NO :38 ou SEQ ID NO :45 ;
(c) la séquence d'acide nucléique selon (a) ou (b), dans laquelle T peut aussi être un U ;
(d) une séquence d'acide nucléique qui est au moins 15, 30, ou 50 bases consécutives en longueur d'une séquence telle que présentée dans SEQ ID NO :21, SEQ ID NO :22, SEQ ID NO :23, SEQ ID NO :26, SEQ ID NO :38 ou SEQ ID NO :45 ;
(e) une sonde d'acide nucléique qui est au moins 15, 30, ou 50 bases consécutives en longueur qui va s'hybrider sélectivement à une séquence d'acide nucléique selon (a) à (d) ;
(f) une séquence d'acide nucléique codant pour une enzyme ayant une activité phosphatase alkaline ayant au moins 70%, 90%, 95%, ou 97% d'identité de séquence avec SEQ ID NO :21, SEQ ID NO :22, SEQ ID NO :23, SEQ ID NO :26, SEQ ID NO :38 ou SEQ ID NO :45 ; et
(g) une séquence d'acide nucléique complémentaire d'une séquence d'acide nucléique selon (a) à (f) ;
incluant facultativement une séquence codante et/ou non-codante.

2. Polynucléotide selon la revendication 1, le polynucléotide étant de l'ADN.

3. Polynucléotide selon la revendication 1, le polynucléotide étant de l'ARN.

4. Polynucléotide comprenant un membre sélectionné parmi le groupe constitué de :
(a) un polynucléotide codant pour une enzyme ayant une activité phosphatase alkaline et ayant au moins 70% d'identité avec un polynucléotide codant pour une enzyme codée par l'ADN contenu dans le dépôt ATCC No. 97536, ladite enzyme étant sélectionnée parmi le groupe constitué de Thermococcus AEDII12RA, Thermococcus celer, Thermococcus CL-2 et Thermococcus GU5L5 ;
(b) un polynucléotide complémentaire du polynucléotide selon (a) ; et
(c) un polynucléotide comprenant au moins 15 bases du polynucléotide selon (a) et (b).

5. Vecteur comprenant un polynucléotide selon l'une quelconque des revendications 1 à 4.

6. Cellule hôte comprenant le vecteur selon la revendication 5.

7. Procédé de production d'un polypeptide comprenant : l'expression, à partir d'une cellule hôte selon la revendication 6, d'un polypeptide codé par ledit ADN et l'isolement du polypeptide.

8. Procédé pour produire une cellule recombinante comprenant : la transformation ou transfection de la cellule avec un vecteur selon la revendication 5, de sorte que la cellule exprime le polypeptide codé par l'ADN contenu dans le vecteur.

9. Enzyme dont au moins une partie est codée par un polynucléotide sélectionné parmi le groupe constitué de :
(a) une séquence d'acide aminé telle que présentée dans SEQ ID NO :30, SEQ ID NO :31, SEQ ID NO :32, SEQ ID NO :35 ou SEQ ID NO :46 ;
(b) une séquence d'acide aminé codée par un polynucléotide selon la revendication 1 ou 4 ;
(c) une séquence d'acide aminé d'au moins 30 acides aminés consécutifs en longueur d'une séquence telle que présentée dans SEQ ID NO :30, SEQ ID NO :31, SEQ ID NO :32, SEQ ID NO :35 ou SEQ ID NO :46 ou un polypeptide codé par le polynucléotide selon la revendication 1 ;
(d) une séquence d'acide aminé ayant une activité phosphatase alkaline et ayant au moins 70%, 90%, 95%, ou 97% d'identité de séquence avec SEQ ID NO :30, SEQ ID NO :31, SEQ ID NO :32, SEQ ID NO :35 ou SEQ ID NO :46 ou un polypeptide codé par le polynucléotide selon la revendication 1.

10. Méthode pour hydrolyser une liaison phosphate comprenant : l'administration d'une quantité efficace d'une enzyme sélectionnée parmi le groupe constitué d'une enzyme ayant une séquence d'acide aminé sélectionnée parmi le groupe de séquences d'acide aminé telles que présentées dans SEQ ID NO :30, 31, 32, 35 ou 46, une enzyme selon la revendication 9, ou une enzyme codée par un polynucléotide selon l'une quelconque des revendications 1 à 4.

11. Phosphatase alkaline thermostable ayant une séquence telle que présentée dans la revendication 9 ou codée par un polynucléotide selon la revendication 1 (a), (b), (c) ou (f).

12. Anticorps généré contre l'enzyme selon la revendication 9 ou généré contre un fragment de l'enzyme qui se lie aux enzymes natives entières.


### SEQUENCE LISTING

(1) GENERAL INFORMATION:
   (i) APPLICANT: RECOMBINANT BIOCATALYSIS, INC.
   (ii) TITLE OF INVENTION: THERMOSTABLE PHOSPHATASES
   (iii) NUMBER OF SEQUENCES: 54
   (iv) CORRESPONDENCE ADDRESS:
      (A) ADDRESSEE:FISH & RICHARDSON
      (B) STREET: 4225 EXECUTIVE SQUARE, STE. 1400
      (C) CITY: LA JOLLA
      (D) STATE: CA
      (E) COUNTRY: USA
      (F) ZIP: 92037
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 6.0
   (vi) CURRENT APPLICATION DATA:
      (A) APPLICATION NUMBER: Unassigned
      (B) FILING DATE: June 19, 1997
      (C) CLASSIFICATION: Unassigned
   (vii) PRIOR APPLICATION DATA:
      (A) APPLICATION NUMBER:
      (B) FILING DATE:
      (C) CLASSIFICATION:
   (viii) ATTORNEY/AGENT INFORMATION:
      (A) NAME: Haile, Lisa A.
      (B) REGISTRATION NUMBER: 38,347
      (C) REFERENCE/DOCKET NUMBER: 09010/015WO1
   (ix) TELECOMMUNICATION INFORMATION:
      (A) TELEPHONE: 619-678-5070
      (B) TELEFAX: 619-678-5099
(2) INFORMATION FOR SEQ ID NO:1:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   CCGAGAATTC ATTAAAGAGG AGAAATTAAC TATGGGGGCA GGTCCGAAAA GG 52
(2) INFORMATION FOR SEQ ID NO:2:
   (i) SEQUENCE CHARACTERISTICS
      (A) LENGTH: 31 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:2:
   CCGAGGATCC TCACCGCCCC CTGCGGGTGC G 31
(2) INFORMATION FOR SEQ ID NO:3:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:3:
   CCGAGAATTC ATTAAAGAGG AGAAATTAAC TATGTTGGAT ATACTGCTTG TT 52
(2) INFORMATION FOR SEQ ID NO:4:
   (i) SEQUENCE CHARACTERISTICS
      (A) LENGTH: 32 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:4:
   CCGAGGATCC TTATTTTTTA ACCAAATTTC CC 32
(2) INFORMATION FOR SEQ ID NO:5:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:5:
   CCGACAATTG ATTAAAGAGG AGAAATTAAC TATGATGATG GAATTCACTC GC 52
(2) INFORMATION FOR SEQ ID NO:6:
   (i) SEQUENCE CHARACTERISTICS
      (A) LENGTH: 32 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:6:
   CGGAGGATCC CTACAGTTCT AAAAGTCTTT TA 32
(2) INFORMATION FOR SEQ ID NO:7:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:7:
   CCGACAATTG ATTAAAGAGG AGAAATTAAC TATGAGAACC CTGACAATAA AC 52
(2) INFORMATION FOR SEQ ID NO:8:
   (i) SEQUENCE CHARACTERISTICS
      (A) LENGTH: 31 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:8:
   CCGAGGATCC TTACACCCAC AGAACCCTTA C 31
(2) INFORMATION FOR SEQ ID NO:9:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:9:
   CCGAGAATTC ATTAAAGAGG AGAAATTAAC TATGAAAGGA AAGTCTCTTG TT 52
(2) INFORMATION FOR SEQ ID NO:10:
   (i) SEQUENCE CHARACTERISTICS
      (A) LENGTH: 31 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:10:
   CCGAGGATCC TCAAGCTTCC TGGAGAATCA A 31
(2) INFORMATION FOR SEQ ID NO:11:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:11:
   CCGAGAATTC ATTAAAGAGG AGAAATTAAC TATGCCAAGA AATATCGCCG CT 52
(2) INFORMATION FOR SEQ ID NO:12:
   (i) SEQUENCE CHARACTERISTICS
      (A) LENGTH: 34 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:12:
   CGGAGGATCC TTAAGGCTTC TCGAGGTGGG GGTT 34
(2) INFORMATION FOR SEQ ID NO:13:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:13:
(2) INFORMATION FOR SEQ ID NO:14:
   (i) SEQUENCE CHARACTERISTICS
      (A) LENGTH: 34 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:14:
   CCGAGGATCC CTAAACATAG TCTAAGTAAT TAGC 34
(2) INFORMATION FOR SEQ ID NO:15:
   (i) SEQUENCE CHARACTERISTICS
      (A) LENGTH: 52 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:15:
(2) INFORMATION FOR SEQ ID NO:16:
   (i) SEQUENCE CHARACTERISTICS
      (A) LENGTH: 31 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:16:
   CCGAGGATCC TCACAGGCTC AGAAGCCTTT G 31
(2) INFORMATION FOR SEQ ID NO:17:
   (i) SEQUENCE CHARACTERISTICS
      (A) LENGTH: 54 NUCLEOTIDES
      (B) TYPE: NUCLEIC ACID
      (C) STRANDEDNESS: SINGLE
      (D) TOPOLOGY: LINEAR
   (ii) MOLECULE TYPE: GENOMIC DNA
   (xi) SEQUENCE DESCRIPTION: SEQ ID NO:17:
   CCGAGAATTC ATTAAAGAGG AGAAATTAAC TATGGAAAAC TTAAAAAAGT ACCT 54
(2) INFORMATION FOR SEQ ID NO:18:
   (i) SEQUENCE CHARACTERISTICS
      (A) LENGTH: 31 NUCLEOTIDES
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:18:
   CGGAAGATCT TCACACCGCC ACTTCCATAT A 31
(2) INFORMATION FOR SEQ ID NO:19:
   (i) SEQUENCE CHARACTERISTICS
      (A) LENGTH: 783 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:19:
   ATG AGG GGG AGC GGA GTG CGG ATA CTT CTC ACC AAC GAT GAC GGC ATC 48
   TTT GCC GAG GGT CTG GGG GCT CTG CGC AAG ATG CTG GAG CCC GTG GCT 96
   ACC CTT TAC GTG GTG GCT CCG GAC CGA GAG CGT AGC GCG GCC AGC CAT 144
   GCT ATC ACC GTT CAC CGC CCC CTG CGG GTG CGG GAG GCG GGT TTT CGC 192
   AGC CCC AGG CTT AAA GGC TGG GTA GTG GAC GGT ACC CCG GCC GAC TGC 240
   GTC AAG CTG GGC CTG GAG GTA CTT TTG CCC GAA CGT CCA GAT TTC CTG 288
   GTT TCG GGC ATA AAC TAC GGG CCC AAC CTG GGT ACC GAC GTA CTT TAC 336
   TCC GGC ACC GTC TCG GCG GCC ATA GAA GGG GTA ATT AAC GGC ATT CCC 384
   TCG GTG GCC GTA TCT TTG GCC ACG CGG CGG GAG CCG GAC TAT ACC TGG 432
   GCG GCC CGG TTC GTC CTG GTC CTG CTG GAG GAA CTG CGA AAA CAC CAA 480
   CTG CCC CCA GGA ACC CTG CTC AAC GTC AAC GTG CCC GAC GGG GTG CCC 528
   CGC GGG GTC AAG GTG ACC AAA CTG GGA AGC GTA CGC TAC GTC AAC GTG 576
   GTA GAC TGC CGC ACC GAC CCT CGG GGG AAG GCT TAC TAC TGG ATG GCG 624
   GGA GAA CCA TTG GAG CTG GAC GGC AAC GAC TCC GAA ACC GAC GTC TGG 672
   GCG GTG CGA GAA GGC TAT ATT TCC GTA ACA CCG GTC CAG ATC GAC CTT 720
   ACT AAC TAC GGC TTC CTG GAA GAA CTC AAA AAA TGG CGT TTC AAG GAT 768
   ATC TTT TCT TCT TAA 783
(2) INFORMATION FOR SEQ ID NO:20:
   (i) SEQUENCE CHARACTERISTICS
      (A) LENGTH: 765 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:20:
(2) INFORMATION FOR SEQ ID NO:21:
   (i) SEQUENCE CHARACTERISTICS
      (A) LENGTH: 765 NUCLEOTIDES
      (B) TYPE: NUCLEIC ACID
      (C) STRANDEDNESS: SINGLE
      ( D) TOPOLOGY: LINEAR
   (ii) MOLECULE TYPE: genomic DNA
   (xi) SEQUENCE DESCRIPTION: SEQ ID NO:21:
   ATG ATG ATG GAA TTC ACT CGC GAG GGA ATA AAA GCT GCT GTA GAG GCA    48
   CTT CAA GGG TTA GGA GAG ATC TAC GTA GTT GCC CCA ATG TTT CAA AGG    96
   AGC GCA AGT GGA AGG GCA ATG ACC ATC CAC AGA CCT CTA AGG GCT AAA   144
   AGA ATA AGT ATG AAC GGT GCA AAA GCA GCC TAT GCT TTG GAT GGA ATG   192
   CCC GTT GAT TGC GTT ATC TTT GCC ATG GCC AGA TTT GGA GAT TTC GAC   240
   CTT GCA ATA AGT GGT GTA AAC TTG GGA GAA AAC ATG AGC ACC GAG ATA   288
   ACG GTT TCC GGG ACT GCA AGC GCT GCA ATA GAG GCT GCA ACC CAA GAG   336
   ATC CCA AGC ATT CCC ATA AGC CTG GAA GTT AAT AGA GAA AAA CAC AAA   384
   TTT GGT GAG GGC GAA GAG ATT GAC TTC TCA GCT GCC AAG TAT TTC CTA   432
   AGA AAA ATC GCA ACG GCG GTT TTA AAG AGA GGC CTC CCC AAA GGA GTC   480
   GAT ATG CTG AAC GTC AAC GTC CCT TAT GAT GCA AAT GAA AGG ACA GAG   528
   ATA GCT TTT ACT CGC CTG GCA AGA AGG ATG TAT AGG CCT TCT ATT GAA   576
   GAG CGC ATA GAC CCA AAG GGG AAT CCC TAC TAC TGG ATA GTT GGA ACT   624
   CAG TGC CCT AAG GAG GCA TTA GAG CCG GGA ACG GAT ATG TAT GTA GTT   672
   AAA GTT GAG AGA AAA GTT AGC GTG ACT CCA ATA AAC ATT GAT ATG ACA   720
   GCA AGA GTG AAT TTA GAC GAG ATT AAA AGA CTT TTA GAA CTG TAG       765
(2) INFORMATION FOR SEQ ID NO:22:
   (i) SEQUENCE CHARACTERISTICS
      (A) LENGTH: 816 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:22:
   ATG AGA ACC CTG ACA ATA AAC ACT GAC GCG GAG GGG TTC GTT TTG AGG    48
   ATT CTC CTG ACG AAC GAC GAT GGA ATC TAC TCC AAC GGA CTG CGC GCC 96
   GCT GTG AAA GCC CTG AGT GAG CTC GGC GAA GTT TAC GTC GTT GCC CCC 144
   CTC TTC CAG AGG AGC GCG AGC GGC AGG GCC ATG ACG CTC CAC AGG CCG 192
   ATA AGG GCC AAG CGC GTT GAC GTT CCC GGC GCA AAG ATA GCC TAC GGA 240
   ATA GAT GGA ACT CCT ACT GAC TGC GTG ATT TTC GCC ATA GCC CGC TTC 288
   GGG AGC TTT GGT TTA GCC GTG AGC GGG ATT AAC CTC GGC GAG AAC CTG 336
   AGC ACC GAG ATA ACA GTC TCA GGG ACG GCC TCC GCT GCC ATA GAG GCC 384
   TCA ACT CAT GGA ATT CCG AGC ATA GCG ATT AGC CTT GAG GTG GAG TGG 432
   AAG AAG ACC CTC GGC GAG GGT GAG GGG GTT GAC TTC TCG GTC TCG ACT 480
   CAC TTC CTC AAG AGA ATC GCG GGA GCC CTC TTG GAG AGA GGT CTT CCT 528
   GAG GGC GTT GAC ATG CTC AAC GTC AAC GTT CCG AGC GAC GCG ACG GAG 576
   GAA ACG GAG ATA GCA ATC ACC CGC TTA GCC CGG AAG CGC TAC TCC CCA 624
   ACG GTC GAG GAG AGG ATT GAC CCC AAG GGC AAC CCC TAC TAC TGG ATT 672
   GTC GGC AAA CTT GTC CAA GAC TTC GAG CCA GGG ACA GAT GCC TAC GCC 720
   CTG AAG GTC GAG AGG AAG GTC AGC GTC ACG CCG ATA AAC ATA GAT ATG 768
   ACT GCG AGG GTG GAC TTT GAG GAG CTT GTA AGG GTT CTG TGG GTG TAA 816
(2) INFORMATION FOR SEQ ID NO:23:
   (i) SEQUENCE CHARACTERISTICS
      (A) LENGTH: 1494 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:23:
   ATG AAA GGA AAG TCT CTT GTT AGC GGT CTG TTG TTG GGT CTT TTA ATT 48
   TTG AGC CTG ATT TCA TTC CAG CCA AGC TTT GCA TAC TCC CCA CAC GGC 96
   GGT GTC AAA AAC ATC ATA ATC CTG GTT GGA GAC GGC ATG GGT CTT GGG 144
   CAT GTA GAA ATT ACA AAG CTC GTT TAT GGA CAC TTA AAC ATG GAA AAC 192
   TTT CCA GTT ACT GGA TTT GAG CTT ACT GAT TCC CTA AGT GGT GAA GTT 240
   ACA GAT TCT GCT GCG GCA GGA ACT GCA ATA TCC ACT GGA GCT AAA ACG 288
   TAT AAT GGT ATG ATT TCA GTA ACC AAC ATA ACC GGA AAG ATA GTT AAC 336
   TTA ACA ACC CTA CTT GAA GTG GCT CAA GAG CTT GGG AAG TCA ACA GGG 384
   CTG GTC ACC ACA ACA AGG ATT ACC CAT GCA ACT CCA GCA GTT TTT GCG 432
   TCC CAT GTC CCA GAT AGG GAT ATG GAG GGG GAG ATA CCC AAG CAA CTC    480
   ATA ATG CAC AAA GTT AAC GTC TTG TTG GGT GGT GGA AGG GAG AAA TTC    528
   GAT GAG AAA AAT TTG GAG CTG GCC AAA AAG CAG GGA TAC AAA GTA GTT    576
   TTC ACG AAG GAA GAG CTT GAA AAA GTT GAA GGA GAT TAT GTC CTA GGA    624
   CTC TTT GCA GAA AGT CAC ATC CCT TAC GTA TTG GAT AGA AAA CCC GAT    672
   GAT GTT GGA CTT TTA GAA ATG GCC AAA AAG GCA ATT TCA ATA CTC GAG    720
   AAG AAC CCG AGC GGA TTC TTT CTC ATG GTT GAG GGC GGA AGG ATT GAC    768
   CAT GCA GCC CAT GGA AAC GAT GTC GCA TCG GTT GTT GCA GAA ACT AAG    816
   GAG TTT GAC GAT GTT GTC AGA TAC GTG CTG GAA TAT CCG AAG AAG AGG    864
   GGA GAT ACC TTG GTA ATA GTG CTT GCC GAT CAC GAA ACT GGA GGT CTT    912
   GCA ATA GGT CTA ACG TAT GGA AAT GCA ATC GAT GAA GAT GCC ATA AGA    960
   AAA ATA AAA GCA AGC ACG TTG AGG ATG CCC AAA GAG GTT AAG GCA GGG   1008
   AGT AGT GTA AAA GAG TCC TCA AAG GTA TGC CGG ATT TGT CCC AAC AGA   1056
   GGA AGA AGT CAG TAT ATT GAG AAT GCG CTG CAC TCG ACA AAC AAG TAT   1104
   GCC CTC TCA AAT GCA GTA GCC GAT GTT ATA AAC AGG CGT ATT GGT GTT   1152
   GGA TTC ACC TCC TAT GAG CAT ACA GGA GTT CCA GTT CCG CTC TTA GCT   1200
   TAC GGT CCC GGG GCA GAG AAC TTC AGA GGT TTC TTA CAC CAT GTG GAT   1248
   ACA GCA AGA TTA GTT GCA AAG TTA ATG CTC TTT GGA AGG AGG AAT ATT   1296
   CCA GTT ACC ATT TCA AGC GTG AGC AGT GTT AAG GGA GAC ATA ACC GGT   1344
   GAT TAC AGG GTT GAT GAG AAG GAT GCC TAC GTT ACG CTC ATG ATG TTT   1392
   CTC GGA GAA AAA GTG GAT AAT GAA ATT GAA AAG AGA GTC GAT ATA GAC   1440
   AAC AAC GGC ATG GTT GAC TTA AAT GAC GTC ATG TTG ATT CTC CAG GAA   1488
   GCT TGA                                                           1494
(2) INFORMATION FOR SEQ ID NO:24:
   (i) SEQUENCE CHARACTERISTICS
      (A) LENGTH: 1755 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:24:
   ATG CCA AGA AAT ATC GCC GCT GTA TGC GCC CTG GCC GCT TTG TTA GGG 48
   TCG GCC TGG GCG GCC AAA GTT GCC GTC TAC CCC TAC GAC GGA GCC GCT 96
   TTG CTG GCG GGG CAG CGC TTC GAT TTG CGC ATA GAA GCC TCC GAG CTG 144
   AAA GGC AAT TTA AAG GCT TAC CGC ATC ACC CTG GAC GGC CAG CCT CTG 192
   GCG GGC CTC GAG CAA ACC GCG CAG GGG GCC GGG CAG GCC GAG TGG ACC 240
   CTG CGC GGT GCC TTC CTG CGC CCT GGA AGC CAC ACC CTC GAG GTC AGC 288
   CTC ACC GAC GAC GCT GGG GAG AGC AGG AAG AGC GTA CGT TGG GAG GCT 336
   CGG CAG AAC CTT CGC TTG CCC CGA GCG GCC AAG AAT GTG ATT CTC TTC 384
   ATT GGC GAC GGG ATG GGC TGG AAC ACC CTC AAC GCC GCC CGC ATC ATC 432
   GCC AAA GGC TTT AAC CCC GAA AAC GGT ATG CCC AAC GGA AAC CTC GAG 480
   ATC GAG AGT GGT TAC GGT GGG ATG GCT ACC GTC ACT ACC GGC AGC TTT 528
   GAT AGC TTC ATC GCC GAC TCA GCT AAC TCG GCT TCT TCC ATC ATG ACC 576
   GGG CAG AAG GTG CAG GTG AAT GCC CTC AAC GTT TAC CCA TCA AAC CTC 624
   AAA GAT ACC CTG GCC TAC CCC CGG ATC GAA ACC CTA GCG GAG ATG CTC 672
   AAG CGG GTA CGC GGG GCC AGC ATT GGG GTA GTG ACC ACC ACC TTC GGC 720
   ACC GAC GCT ACC CCG GCT TCA CTC AAC GCC CAT ACC CGC CGC CGC GGT 768
   GAT TAC CAG GCT ATC GCC GAC ATG TAC TTT GGT AGA GGC GGG TTC GGT 816
   GTT CCC TTG GAT GTG ATG CTC TTC GGT GGT TCA CGC GAC TTC ATC CCC 864
   CAG AGC ACC CCT GGC TCG CGG CGC AAG GAT AGC ACG GAC TGG ATT GCC 912
   GAA TCC CAG AAG CTG GGC TAC ACC TTT GTC AGC ACC CGC AGC GAG CTG 960
   CTG GCG GCC AAA CCC ACC GAT AAG CTG TTT GGG CTG TTC AAC ATT GAC 1008
   AAC TTC CCC AGC TAC CTA GAC CGC GCA GTG TGG AAG CGG CCC GAG ATG 1056
   CTG GGA AGC TTT ACC GAT ATG CCC TAC CTC TGG GAG ATG ACC CAG AAA 1104
   GCC GTG GAG GCT CTC TCC AGA AAC GAC AAA GGC TTT TTC TTG ATG GTT 1152
   GAG GGG GGA ATG GTG GAT AAG TAC GAG CAC CCC TTG GAC TGG CCC CGC 1200
   GCA CTT TGG GAT GTA CTC GAG CTG GAC CGC GCG GTG GCT TGG GCC AAG 1248
   GGC TAT GCG GCC TCC CAC CCC GAT ACC CTG GTG ATT GTC ACC GCC GAC 1296
   CAC GCT CAC TCG ATC TCG GTG TTT GGC GGT TAC GAC TAC TCC AAG CAG 1344
   GGC CGG GAG GGG GTG GGG GTT TAT GAG GCC GCC AAG TTC CCC ACC TAC 1392
   GGC GAC AAA AAA GAC GCC AAC GGC TTT CCC TTG CCC GAC ACC ACT CGG 1440
   GGA ATC GCG GTA GGC TTC GGG GCC ACG CCG GAT TAC TGT GAA ACC TAC 1488
   CGG GGC CGC GAG GTC TAC AAA GAC CCC ACC ATC TCC GAC GGC AAA GGT 1536
   GGT TAC GTG GCC AAC CCT GAG GTC TGC AAG GAG CCG GGC CTT CCA ACG 1584
   TAC CGG CAA CTC CCA GTA GAT AGC GCC CAG GGC GTG CAC ACG GCT GAT 1632
   CCC ATG CCG CTG TTT GCC TTT GGC GTG GGG TCT CAG TTC TTC AAT GGC 1680
   CTC ATC GAC CAG ACC GAG ATC TTC TTC CGC ATG GCC CAG GCC CTA GGG 1728
   TTC AAC CCC CAC CTC GAG AAG CCT TAA 1755
(2) INFORMATION FOR SEQ ID NO:25:
   (i) SEQUENCE CHARACTERISTICS
      (A) LENGTH: 912 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:25:
(2) INFORMATION FOR SEQ ID NO:26:
   (i) SEQUENCE CHARACTERISTICS
      (A) LENGTH: 774 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:26:
   ATG AGA ATC CTC CTC ACC AAC GAC GAC GGC ATC TAT TCC AAC GGT CTG    48
   CGC GCG GCG GTG AAG GGC CTG AGC GAG CTC GGC GAG GTC TAC GTC GTC    96
   GCC CCG CTC TTC CAG AGG AGC GCG AGC GGT CGG GCG ATG ACC CTA CAC   144
   AGG CCG ATA AGG GCA AAG AGG GTT GAC GTT CCC GGC GCG AAG ATA GCG   192
   TAT GGC ATA GAC GGA ACG CCG ACC GAC TGC GTG ATT TTT GCC ATC GCC   240
   CGC TTC GGC GAC TTT GAT CTG GCG GTC AGC GGG ATA AAC CTA GGC GAG   288
   AAC CTG AGC ACG GAG ATA ACC GTC TCC GGA ACG GCC TCG GCG GCG ATA   336
   GAG GCT TCC ACC CAC GGG ATT CCA AGT GTA GCT ATA AGC CTC GAG GTC   384
   GAG TGG AAG AAG ACC CTC GGC GAG GGG GAG GGT ATT GAC TTC TCG GTT   432
   TCA GCA CAC TTC CTG AGA AGG ATA GCG ACG GCT GTC CTT AAG AAG GGC   480
   CTG CCT GAA GGG GTG GAC ATG CTC AAC GTG AAC GTC CCT AGC GAC GCC   528
   AGC GAG GGG ACT GAG ATC GCC ATA ACG CGC CTC GCG AGG AAG CGC TAT   576
   TCT CCG ACG ATA GAG GAG AGG ATA GAC CCC AAG GGC AAC CCC TAC TAC   624
   TGG ATC GTT GGC AGG CTC GTC CAG GAG TTC GAG CCG GGC ACG GAC GCC   672
   TAC GCT CTG AAA GTC GAG AGA AAG GTC AGC GTC ACG CCC ATA AAC ATC   720
   GAC ATG ACT GCG AGG GTT GAC TTT GAG AAC CTT CAA AGG CTT CTG AGC   768
   CTG TGA                                                           774
(2) INFORMATION FOR SEQ ID NO:27;
   (i) SEQUENCE CHARACTERISTICS
      (A) LENGTH: 795 NUCLEOTIDES
      (B) TYPE: NUCLEIC ACID
      (C) STRANDEDNESS: SINGLE
      (D) TOPOLOGY: LINEAR
   (ii) MOLECULE TYPE: genomic DNA
   (xi) SEQUENCE DESCRIPTION: SEQ ID NO:27:
   ATG GAA AAC TTA AAA AAG TAC CTA GAA GTT GCA AAA ATA GCC GCG CTC     48
   GCG GGT GGG CAG GTT CTG AAA GAA AAC TTC GGA AAG GTA AAA AAG GAA     96
   AAC ATA GAG GAA AAA GGG GAA AAG GAC TTT GTA AGT TAC GTG GAT AAA    144
   ACT TCA GAG GAA AGG ATA AAG GAG GTG ATA CTC AAG TTC TTT CCC GAT    192
   CAC GAG GTC GTA GGG GAA GAG ATG GGT GCG GAG GGA AGC GGA AGC GAA    240
   TAC AGG TGG TTC ATA GAC CCC CTT GAC GGC ACA AAG AAC TAC ATA AAC    288
   GGT TTT CCC ATC TTT GCC GTA TCA GTG GGA CTT GTT AAG GGA GAA GAG    336
   CCA ATT GTG GGT GCG GTT TAC CTT CCT TAC TTT GAC AAG CTT TAC TGG    384
   GGT GCT AAA GGT CTC GGG GCT TAC GTA AAC GGA AAG AGG ATA AAG GTA    432
   AAG GAC AAT GAG AGT TTA AAG CAC GCC GGA GTG GTT TAC GGA TTT CCC    480
   TCT AGG AGC AGG AGG GAC ATA TCT ATC TAC TTG AAC ATA TTC AAG GAT    528
   GTC TTT TAC GAA GTT GGC TCT ATG AGG AGA CCC GGG GCT GCT GCG GTT    576
   GAC CTC TGC ATG GTG GCG GAA GGG ATA TTT GAC GGG ATG ATG GAG TTT    624
   GAA ATG AAG CCG TGG GAC ATA ACC GCA GGG CTT GTA ATA CTG AAG GAA    672
   GCC GGG GGC GTT TAC ACA CTT GTG GGA GAA CCC TTC GGA GTT TCG GAC    720
   ATA ATT GCG GGC AAC AAA GCC CTC CAC GAC TTT ATA CTT CAG GTA GCC    768
   AAA AAG TAT ATG GAA GTG GCG GTG TGA                                795
(2) INFORMATION FOR SEQ ID NO:28:
   (i) SEQUENCE CHARACTERISTICS
      (A) LENGTH: 260 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:28:
   Met Arg Gly Ser Gly Val Arg Ile Leu Leu Thr Asn Asp Asp Gly Ile
                   5                  10                  15
   Phe Ala Glu Gly Leu Gly Ala Leu Arg Lys Met Leu Glu Pro Val Ala
               20                  25                  30
   Thr Leu Tyr Val Val Ala Pro Asp Arg Glu Arg Ser Ala Ala Ser His
           35                  40                  45
   Ala Ile Thr Val His Arg Pro Leu Arg Val Arg Glu Ala Gly Phe Arg
       50                  55                  60
   Ser Pro Arg Leu Lys Gly Trp Val Val Asp Gly Thr Pro Ala Asp Cys
   65                  70                  75                  80
   Val Lys Leu Gly Leu Glu Val Leu Leu Pro Glu Arg Pro Asp Phe Leu
                   85                  90                  95
   Val Ser Gly Ile Asn Tyr Gly Pro Asn Leu Gly Thr Asp Val Leu Tyr
               100                 105                 110
   Ser Gly Thr Val Ser Ala Ala Ile Glu Gly Val Ile Asn Gly Ile Pro
           115                 120                 125
   Ser Val Ala Val Ser Leu Ala Thr Arg Arg Glu Pro Asp Tyr Thr Trp
       130                 135                 140
   Ala Ala Arg Phe Val Leu Val Leu Leu Glu Glu Leu Arg Lys His Gln
   145                 150                 155                 160
   Leu Pro Pro Gly Thr Leu Leu Asn Val Asn Val Pro Asp Gly Val Pro
                   165                 170                 175
   Arg Gly Val Lys Val Thr Lys Leu Gly Ser Val Arg Tyr Val Asn Val
               180                 185                 190
   Val Asp Cys Arg Thr Asp Pro Arg Gly Lys Ala Tyr Tyr Trp Met Ala
           195                 200                 205
   Gly Glu Pro Leu Glu Leu Asp Gly Asn Asp Ser Glu Thr Asp Val Trp
       210                 215                 220
   Ala Val Arg Glu Gly Tyr Ile Ser Val Thr Pro Val Gln Ile Asp Leu
   225                 230                 235                 240
   Thr Asn Tyr Gly Phe Leu Glu Glu Leu Lys Lys Trp Arg Phe Lys Asp
                   245                 250                 255
   Ile Phe Ser Ser
               260
(2) INFORMATION FOR SEQ ID NO:29:
   (i) SEQUENCE CHARACTERISTICS
      (A) LENGTH: 265 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:29:
   Met Leu Asp Ile Leu Leu Val Asn Asp Asp Gly Ile Tyr Ser Asn Gly
                   5                   10                  15
   Leu Ile Ala Leu Lys Asp Ala Leu Leu Glu Lys Phe Asn Ala Arg Ile
               20                  25                  30
   Thr Ile Val Ala Pro Thr Asn Gln Gln Ser Gly Ile Gly Arg Ala Ile
           35                  40                  45
   Ser Leu Phe Glu Pro Leu Arg Ile Thr Lys Thr Lys Leu Ala Asp Gly
       50                  55                  60
   Ser Trp Gly Tyr Ala Val Ser Gly Thr Pro Thr Asp Cys Val Ile Leu
   65                  70                  75                  80
   Gly Ile Tyr Glu Ile Leu Lys Lys Val Pro Asp Val Val Ile Ser Gly
                   85                  90                  95
   Ile Asn Ile Gly Glu Asn Leu Gly Thr Glu Ile Thr Thr Ser Gly Thr
               100                 105                 110
   Leu Gly Ala Ala Phe Glu Gly Ala His His Gly Ala Lys Ala Leu Ala
           115                 120                 125
   Ser Ser Leu Gln Val Thr Ser Asp His Leu Lys Phe Lys Glu Gly Glu
       130                 135                 140
   Thr Pro Ile Asp Phe Thr Val Pro Ala Arg Ile Thr Ala Asn Val Val
   145                 150                 155                 160
   Glu Lys Met Leu Asp Tyr Asp Phe Pro Cys Asp Val Val Asn Leu Asn
                   165                 170                 175
   Ile Pro Glu Gly Ala Thr Glu Lys Thr Pro Ile Glu Ile Thr Arg Leu
               180                 185                 190
   Ala Arg Lys Met Tyr Thr Thr His Val Glu Glu Arg Ile Asp Pro Arg
           195                 200                 205
   Gly Arg Ser Tyr Tyr Trp Ile Asp Gly Tyr Pro Ile Leu Glu Glu Glu
       210                 215                 220
   Glu Asp Thr Asp Val Tyr Val Val Arg Arg Lys Gly His Ile Ser Leu
   225                 230                 235                 240
   Thr Pro Leu Thr Leu Asp Thr Thr Ile Lys Asn Leu Glu Glu Phe Lys
                   245                 250                 255
   Lys Lys Tyr Glu Arg Ile Leu Asn Glu
               260                 265
(2) INFORMATION FOR SEQ ID NO:30:
   (i) SEQUENCE CHARACTERISTICS
      (A) LENGTH: 254 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:30:
   Met Met Met Glu Phe Thr Arg Glu Gly Ile Lys Ala Ala Val Glu Ala
                    5                  10                  15
   Leu Gln Gly Leu Gly Glu Ile Tyr Val Val Ala Pro Met Phe Gln Arg
               20                  25                  30
   Ser Ala Ser Gly Arg Ala Met Thr Ile His Arg Pro Leu Arg Ala Lys
           35                  40                  45
   Arg Ile Ser Met Asn Gly Ala Lys Ala Ala Tyr Ala Leu Asp Gly Met
       50                  55                  60
   Pro Val Asp Cys Val Ile Phe Ala Met Ala Arg Phe Gly Asp Phe Asp
   65                  70                  75                  80
   Leu Ala Ile Ser Gly Val Asn Leu Gly Glu Asn Met Ser Thr Glu Ile
                   85                  90                  95
   Thr Val Ser Gly Thr Ala Ser Ala Ala Ile Glu Ala Ala Thr Gln Glu
               100                 105                 110
   Ile Pro Ser Ile Pro Ile Ser Leu Glu Val Asn Arg Glu Lys His Lys
           115                 120                 125
   Phe Gly Glu Gly Glu Glu Ile Asp Phe Ser Ala Ala Lys Tyr Phe Leu
       130                 135                 140
   Arg Lys Ile Ala Thr Ala Val Leu Lys Arg Gly Leu Pro Lys Gly Val
   145                 150                 155                 160
   Asp Met Leu Asn Val Asn Val Pro Tyr Asp Ala Asn Glu Arg Thr Glu
                   165                 170                 175
   Ile Ala Phe Thr Arg Leu Ala Arg Arg Met Tyr Arg Pro Ser Ile Glu
               180                 185                 190
   Glu Arg Ile Asp Pro Lys Gly Asn Pro Tyr Tyr Trp Ile Val Gly Thr
           195                 200                 205
   Gln Cys Pro Lys Glu Ala Leu Glu Pro Gly Thr Asp Met Tyr Val Val
       210                 215                 220
   Lys Val Glu Arg Lys Val Ser Val Thr Pro Ile Asn Ile Asp Met Thr
   225                 230                 235                 240
   Ala Arg Val Asn Leu Asp Glu Ile Lys Arg Leu Leu Glu Leu
                   245                 250
(2) INFORMATION FOR SEQ ID NO:31:
   (i) SEQUENCE CHARACTERISTICS
      (A) LENGTH: 271 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:31:
   Met Arg Thr Leu Thr Ile Asn Thr Asp Ala Glu Gly Phe Val Leu Arg
                       5                  10                  15
   Ile Leu Leu Thr Asn Asp Asp Gly Ile Tyr Ser Asn Gly Leu Arg Ala
                   20                  25                  30
   Ala Val Lys Ala Leu Ser Glu Leu Gly Glu Val Tyr Val Val Ala Pro
               35                  40                  45
   Leu Phe Gln Arg Ser Ala Ser Gly Arg Ala Met Thr Leu His Arg Pro
           50                  55                  60
   Ile Arg Ala Lys Arg Val Asp Val Pro Gly Ala Lys Ile Ala Tyr Gly
   65                  70                  75                  80
   Ile Asp Gly Thr Pro Thr Asp Cys Val Ile Phe Ala Ile Ala Arg Phe
                   85                  90                  95
   Gly Ser Phe Gly Leu Ala Val Ser Gly Ile Asn Leu Gly Glu Asn Leu
               100                 105                 110
   Ser Thr Glu Ile Thr Val Ser Gly Thr Ala Ser Ala Ala Ile Glu Ala
           115                 120                 125
   Ser Thr His Gly Ile Pro Ser Ile Ala Ile Ser Leu Glu Val Glu Trp
       130                 135                 140
   Lys Lys Thr Leu Gly Glu Gly Glu Gly Val Asp Phe Ser Val Ser Thr
   145                 150                 155                 160
   His Phe Leu Lys Arg Ile Ala Gly Ala Leu Leu Glu Arg Gly Leu Pro
                   165                 170                 175
   Glu Gly Val Asp Met Leu Asn Val Asn Val Pro Ser Asp Ala Thr Glu
               180                 185                 190
   Glu Thr Glu Ile Ala Ile Thr Arg Leu Ala Arg Lys Arg Tyr Ser Pro
           195                 200                 205
   Thr Val Glu Glu Arg Ile Asp Pro Lys Gly Asn Pro Tyr Tyr Trp Ile
       210                 215                 220
   Val Gly Lys Leu Val Gln Asp Phe Glu Pro Gly Thr Asp Ala Tyr Ala
   225                 230                 235                 240
   Leu Lys Val Glu Arg Lys Val Ser Val Thr Pro Ile Asn Ile Asp Met
                   245                 250                 255
   Thr Ala Arg Val Asp Phe Glu Glu Leu Val Arg Val Leu Trp Val
               260                 265                 270
(2) INFORMATION FOR SEQ ID NO:32:
   (i) SEQUENCE CHARACTERISTICS
      (A) LENGTH: 497 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:32:
   Met Lys Gly Lys Ser Leu Val Ser Gly Leu Leu Leu Gly Leu Leu Ile
                    5                   10                  15
   Leu Ser Leu Ile Ser Phe Gln Pro Ser Phe Ala Tyr Ser Pro His Gly
                20                  25                  30
   Gly Val Lys Asn Ile Ile Ile Leu Val Gly Asp Gly Met Gly Leu Gly
            35                  40                  45
   His Val Glu Ile Thr Lys Leu Val Tyr Gly His Leu Asn Met Glu Asn
        50                  55                  60
   Phe Pro Val Thr Gly Phe Glu Leu Thr Asp Ser Leu Ser Gly Glu Val
    65                  70                  75                      80
   Thr Asp Ser Ala Ala Ala Gly Thr Ala Ile Ser Thr Gly Ala Lys Thr
                    85                  90                  95
   Tyr Asn Gly Met Ile Ser Val Thr Asn Ile Thr Gly Lys Ile Val Asn
               100                 105                 110
   Leu Thr Thr Leu Leu Glu Val Ala Gln Glu Leu Gly Lys Ser Thr Gly
           115                 120                 125
   Leu Val Thr Thr Thr Arg Ile Thr His Ala Thr Pro Ala Val Phe Ala
       130                 135                 140
   Ser His Val Pro Asp Arg Asp Met Glu Gly Glu Ile Pro Lys Gln Leu
   145                 150                 155                 160
   Ile Met His Lys Val Asn Val Leu Leu Gly Gly Gly Arg Glu Lys Phe
                   165                 170                 175
   Asp Glu Lys Asn Leu Glu Leu Ala Lys Lys Gln Gly Tyr Lys Val Val
               180                 185                 190
   Phe Thr Lys Glu Glu Leu Glu Lys Val Glu Gly Asp Tyr Val Leu Gly
           195                 200                 205
   Leu Phe Ala Glu Ser His Ile Pro Tyr Val Leu Asp Arg Lys Pro Asp
       210                 215                 220
   Asp Val Gly Leu Leu Glu Met Ala Lys Lys Ala Ile Ser Ile Leu Glu
   225                 230                 235                 240
   Lys Asn Pro Ser Gly Phe Phe Leu Met Val Glu Gly Gly Arg Ile Asp
                   245                 250                 255
   His Ala Ala His Gly Asn Asp Val Ala Ser Val Val Ala Glu Thr Lys
               260                 265                 270
   Glu Phe Asp Asp Val Val Arg Tyr Val Leu Glu Tyr Pro Lys Lys Arg
           275                 280                 285
   Gly Asp Thr Leu Val Ile Val Leu Ala Asp His Glu Thr Gly Gly Leu
       290                 295                 300
   Ala Ile Gly Leu Thr Tyr Gly Asn Ala Ile Asp Glu Asp Ala Ile Arg
   305                 310                 315                 320
   Lys Ile Lys Ala Ser Thr Leu Arg Met Pro Lys Glu Val Lys Ala Gly
                   325                 330                 335
   Ser Ser Val Lys Glu Ser Ser Lys Val Cys Arg Ile Cys Pro Asn Arg
               340                 345                 350
   Gly Arg Ser Gln Tyr Ile Glu Asn Ala Leu His Ser Thr Asn Lys Tyr
           355                 360                 365
   Ala Leu Ser Asn Ala Val Ala Asp Val Ile Asn Arg Arg Ile Gly Val
       370                 375                 380
   Gly Phe Thr Ser Tyr Glu His Thr Gly Val Pro Val Pro Leu Leu Ala
   385                 390                 395                 400
   Tyr Gly Pro Gly Ala Glu Asn Phe Arg Gly Phe Leu His His Val Asp
                   405                 410                 415
   Thr Ala Arg Leu Val Ala Lys Leu Met Leu Phe Gly Arg Arg Asn Ile
               420                 425                 430
   Pro Val Thr Ile Ser Ser Val Ser Ser Val Lys Gly Asp Ile Thr Gly
           435                 440                 445
   Asp Tyr Arg Val Asp Glu Lys Asp Ala Tyr Val Thr Leu Met Met Phe
       450                 455                 460
   Leu Gly Glu Lys Val Asp Asn Glu Ile Glu Lys Arg Val Asp Ile Asp
   465                 470                 475                 480
   Asn Asn Gly Met Val Asp Leu Asn Asp Val Met Leu Ile Leu Gln Glu
                   485                 490                 495
   Ala
   497
(2) INFORMATION FOR SEQ ID NO:33:
   (i) SEQUENCE CHARACTERISTICS
      (A) LENGTH: 584 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:33:
   Met Pro Arg Asn Ile Ala Ala Val Cys Ala Leu Ala Ala Leu Leu Gly
                   5                   10                  15
   Ser Ala Trp Ala Ala Lys Val Ala Val Tyr Pro Tyr Asp Gly Ala Ala
               20                  25                  30
   Leu Leu Ala Gly Gln Arg Phe Asp Leu Arg Ile Glu Ala Ser Glu Leu
           35                  40                  45
   Lys Gly Asn Leu Lys Ala Tyr Arg Ile Thr Leu Asp Gly Gln Pro Leu
       50                  55                  60
   Ala Gly Leu Glu Gln Thr Ala Gln Gly Ala Gly Gln Ala Glu Trp Thr
   65                  70                  75                  80
   Leu Arg Gly Ala Phe Leu Arg Pro Gly Ser His Thr Leu Glu Val Ser
                   85                  90                  95
   Leu Thr Asp Asp Ala Gly Glu Ser Arg Lys Ser Val Arg Trp Glu Ala
               100                 105                 110
   Arg Gln Asn Leu Arg Leu Pro Arg Ala Ala Lys Asn Val Ile Leu Phe
           115                 120                 125
   Ile Gly Asp Gly Met Gly Trp Asn Thr Leu Asn Ala Ala Arg Ile Ile
       130                 135                 140
   Ala Lys Gly Phe Asn Pro Glu Asn Gly Met Pro Asn Gly Asn Leu Glu
   145                 150                 155                 160
   Ile Glu Ser Gly Tyr Gly Gly Met Ala Thr Val Thr Thr Gly Ser Phe
                   165                 170                 175
   Asp Ser Phe Ile Ala Asp Ser Ala Asn Ser Ala Ser Ser Ile Met Thr
               180                 185                 190
   Gly Gln Lys Val Gln Val Asn Ala Leu Asn Val Tyr Pro Ser Asn Leu
           195                 200                 205
   Lys Asp Thr Leu Ala Tyr Pro Arg Ile Glu Thr Leu Ala Glu Met Leu
       210                 215                 220
   Lys Arg Val Arg Gly Ala Ser Ile Gly Val Val Thr Thr Thr Phe Gly
   225                 230                 235                 240
   Thr Asp Ala Thr Pro Ala Ser Leu Asn Ala His Thr Arg Arg Arg Gly
                   245                 250                 255
   Asp Tyr Gln Ala Ile Ala Asp Met Tyr Phe Gly Arg Gly Gly Phe Gly
               260                 265                 270
   Val Pro Leu Asp Val Met Leu Phe Gly Gly Ser Arg Asp Phe Ile Pro
           275                 280                 285
   Gln Ser Thr Pro Gly Ser Arg Arg Lys Asp Ser Thr Asp Trp Ile Ala
       290                 295                 300
   Glu Ser Gln Lys Leu Gly Tyr Thr Phe Val Ser Thr Arg Ser Glu Leu
   305                 310                 315                 320
   Leu Ala Ala Lys Pro Thr Asp Lys Leu Phe Gly Leu Phe Asn Ile Asp
                   325                 330                 335
   Asn Phe Pro Ser Tyr Leu Asp Arg Ala Val Trp Lys Arg Pro Glu Met
               340                 345                 350
   Leu Gly Ser Phe Thr Asp Met Pro Tyr Leu Trp Glu Met Thr Gln Lys
           355                 360                 365
   Ala Val Glu Ala Leu Ser Arg Asn Asp Lys Gly Phe Phe Leu Met Val
       370                 375                 380
   Glu Gly Gly Met Val Asp Lys Tyr Glu His Pro Leu Asp Trp Pro Arg
   385                 390                 395                 400
   Ala Leu Trp Asp Val Leu Glu Leu Asp Arg Ala Val Ala Trp Ala Lys
                   405                 410                 415
   Gly Tyr Ala Ala Ser His Pro Asp Thr Leu Val Ile Val Thr Ala Asp
               420                 425                 430
   His Ala His Ser Ile Ser Val Phe Gly Gly Tyr Asp Tyr Ser Lys Gln
           435                 440                 445
   Gly Arg Glu Gly Val Gly Val Tyr Glu Ala Ala Lys Phe Pro Thr Tyr
       450                 455                 460
   Gly Asp Lys Lys Asp Ala Asn Gly Phe Pro Leu Pro Asp Thr Thr Arg
   465                 470                 475                 480
   Gly Ile Ala Val Gly Phe Gly Ala Thr Pro Asp Tyr Cys Glu Thr Tyr
                   485                 490                 495
   Arg Gly Arg Glu Val Tyr Lys Asp Pro Thr Ile Ser Asp Gly Lys Gly
               500                 505                 510
   Gly Tyr Val Ala Asn Pro Glu Val Cys Lys Glu Pro Gly Leu Pro Thr
           515                 520                 525
   Tyr Arg Gln Leu Pro Val Asp Ser Ala Gln Gly Val His Thr Ala Asp
       530                 535                 540
   Pro Met Pro Leu Phe Ala Phe Gly Val Gly Ser Gln Phe Phe Asn Gly
   545                 550                 555                 560
   Leu Ile Asp Gln Thr Glu Ile Phe Phe Arg Met Ala Gln Ala Leu Gly
                   565                 570                 575
   Phe Asn Pro His Leu Glu Lys Pro
               580
(2) INFORMATION FOR SEQ ID NO:34:
   (i) SEQUENCE CHARACTERISTICS
      (A) LENGTH: 301 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:34:
   Met Tyr Lys Trp Ile Ile Glu Gly Lys Leu Ala Gln Ala Pro Phe Pro
                   5                   10                  15
   Ser Leu Gly Glu Leu Ala Asp Leu Lys Arg Leu Phe Asp Ala Ile Ile
               20                  25                  30
   Val Leu Thr Met Pro His Glu Gln Pro Leu Asn Glu Lys Tyr Ile Glu
           35                  40                  45
   Ile Leu Glu Ser His Gly Phe Gln Val Leu His Val Pro Thr Leu Asp
       50                  55                  60
   Phe His Pro Leu Glu Leu Phe Asp Leu Leu Lys Thr Ser Ile Phe Ile
   65                  70                  75                  80
   Asp Glu Asn Leu Glu Arg Ser His Arg Val Leu Val His Cys Met Gly
                   85                  90                  95
   Gly Ile Gly Arg Ser Gly Leu Val Thr Ala Ala Tyr Leu Ile Phe Lys
               100                 105                 110
   Gly Tyr Asp Ile Tyr Asp Ala Val Lys His Val Arg Thr Val Val Pro
           115                 120                 125
   Gly Ala Ile Glu Asn Arg Gly Gln Ala Leu Met Leu Glu Asn Tyr Tyr
       130                 135                 140
   Thr Leu Val Lys Ser Phe Asn Arg Glu Leu Leu Arg Asp Tyr Gly Lys
   145                 150                 155                 160
   Lys Ile Phe Thr Leu Gly Asp Pro Lys Ala Val Leu His Ala Ser Lys
                   165                 170                 175
   Thr Thr Gln Phe Thr Ile Glu Leu Leu Ser Asn Leu His Val Asn Glu
               180                 185                 190
   Ala Phe Ser Ile Ser Ala Met Ala Gln Ser Leu Leu His Phe His Asp
           195                 200                 205
   Val Lys Val Arg Ser Lys Leu Lys Glu Val Phe Glu Asn Met Glu Phe
       210                 215                 220
   Ser Ser Ala Ser Glu Glu Val Leu Ser Phe Ile His Leu Leu Asp Phe
   225                 230                 235                 240
   Tyr Gln Asp Gly Arg Val Val Leu Thr Ile Tyr Asp Tyr Leu Pro Asp
                   245                 250                 255
   Arg Val Asp Leu Ile Leu Leu Cys Lys Trp Gly Cys Asp Lys Ile Val
               260                 265                 270
   Glu Val Ser Ser Ser Ala Lys Lys Thr Val Glu Lys Leu Val Gly Arg
               275                 280                 285
   Lys Val Ser Leu Ser Trp Ala Asn Tyr Leu Asp Tyr Val
       290                 295                 300
(2) INFORMATION FOR SEQ ID NO:35:
   (i) SEQUENCE CHARACTERISTICS
      (A) LENGTH: 257 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:35:
   Met Arg Ile Leu Leu Thr Asn Asp Asp Gly Ile Tyr Ser Asn Gly Leu
                   5                   10                  15
   Arg Ala Ala Val Lys Gly Leu Ser Glu Leu Gly Glu Val Tyr Val Val
               20                  25                  30
   Ala Pro Leu Phe Gln Arg Ser Ala Ser Gly Arg Ala Met Thr Leu His
           35                  40                  45
   Arg Pro Ile Arg Ala Lys Arg Val Asp Val Pro Gly Ala Lys Ile Ala
       50                  55                  60
   Tyr Gly Ile Asp Gly Thr Pro Thr Asp Cys Val Ile Phe Ala Ile Ala
   65                  70                  75                  80
   Arg Phe Gly Asp Phe Asp Leu Ala Val Ser Gly Ile Asn Leu Gly Glu
                   85                  90                  95
   Asn Leu Ser Thr Glu Ile Thr Val Ser Gly Thr Ala Ser Ala Ala Ile
               100                 105                 110
   Glu Ala Ser Thr His Gly Ile Pro Ser Val Ala Ile Ser Leu Glu Val
           115                 120                 125
   Glu Trp Lys Lys Thr Leu Gly Glu Gly Glu Gly Ile Asp Phe Ser Val
       130                 135                 140
   Ser Ala His Phe Leu Arg Arg Ile Ala Thr Ala Val Leu Lys Lys Gly
   145                 150                 155                 160
   Leu Pro Glu Gly Val Asp Met Leu Asn Val Asn Val Pro Ser Asp Ala
                   165                 170                 175
   Ser Glu Gly Thr Glu Ile Ala Ile Thr Arg Leu Ala Arg Lys Arg Tyr
               180                 185                 190
   Ser Pro Thr Ile Glu Glu Arg Ile Asp Pro Lys Gly Asn Pro Tyr Tyr
           195                 200                 205
   Trp Ile Val Gly Arg Leu Val Gln Glu Phe Glu Pro Gly Thr Asp Ala
       210                 215                 220
   Tyr Ala Leu Lys Val Glu Arg Lys Val Ser Val Thr Pro Ile Asn Ile
   225                 230                 235                 240
   Asp Met Thr Ala Arg Val Asp Phe Glu Asn Leu Gln Arg Leu Leu Ser
                   245                 250                 255
   Leu
(2) INFORMATION FOR SEQ ID NO:36:
   (i) SEQUENCE CHARACTERISTICS
      (A) LENGTH: 264 AMINO ACIDS
      (B) TYPE: AMINO ACID
      (D) TOPOLOGY: LINEAR
   (ii) MOLECULE TYPE: PROTEIN
   (xi) SEQUENCE DESCRIPTION: SEQ ID NO:36: